(19) Europäisches Patentamt
European Patent Office
Office européen des brevets

(11) Veröffentlichungsnummer : **0 235 628 B1**

(12) **EUROPÄISCHE PATENTSCHRIFT**

(45) Veröffentlichungstag der Patentschrift : **24.04.91 Patentblatt 91/17**

(21) Anmeldenummer : **87101902.2**

(22) Anmeldetag : **11.02.87**

(51) Int. Cl.$^5$ : **C07D 231/44**, C07D 401/04, A01N 47/02, A01N 43/56, // C07D231/18, C07D231/38

# (54) 1-Arylpyrazole.

(30) Priorität : **28.02.86 DE 3606476**

(43) Veröffentlichungstag der Anmeldung : **09.09.87 Patentblatt 87/37**

(45) Bekanntmachung des Hinweises auf die Patenterteilung : **24.04.91 Patentblatt 91/17**

(84) Benannte Vertragsstaaten : **AT BE CH DE ES FR GB IT LI NL**

(56) Entgegenhaltungen :
**EP-A- 0 154 115**
**EP-A- 0 201 852**
**IL FARMACO ED. SCIENTIFICA, Band 26, Nr. 4, April 1971, Seiten 276-293; P. GIORI et al.: "Sintesi ed attività antifungina di solfuri pirazolici"**
**IL FARMACO ED. SCIENTIFICA, Band 38, Nr. 4, April 1983, Seiten 274-282; P. GIORI et al.: "Synthesis and antifungal activity of pyrazole derivatives containing sulfurated functions"**
**CHEMICAL ABSTRACTS, Band 89, Nr. 21, 20. November 1978, Seite 604, Spalte 1, Zusammenfassungsnr. 179950x, Columbus, Ohio, US; V.M. NEPLYNEV et al.: "Synthesis of pyrazole, isoxazole, and pyrimidine derivatives based on 2-arylsulforyl-2-cyanovinyl ethyl ethers"**
**PATENT ABSTRACTS OF JAPAN, Band 5, Nr. 124 (C-66)[796], 11. August 1981; & JP - A - 56 59760**

(73) Patentinhaber : **BAYER AG**
**W-5090 Leverkusen 1 Bayerwerk (DE)**

(72) Erfinder : **Gehring, Reinhold, Dr.**
**Dasnöckel 49**
**W-5600 Wuppertal 1 (DE)**
Erfinder : **Jensen-Korte, Uta Dr.**
**Geibelstrasse 9**
**W-4000 Düsseldorf 1 (DE)**
Erfinder : **Schallner, Otto Dr.**
**Noldeweg 22**
**W-4019 Monheim (DE)**
Erfinder : **Stetter, Jörg, Dr.**
**Gellertweg 4**
**W-5600 Wuppertal 1 (DE)**
Erfinder : **Wroblowsky, Heinz-Jürgen, Dr.**
**Gladbacher Strasse 34**
**W-4018 Langenfeld (DE)**
Erfinder : **Becker, Benedikt, Dr.**
**Metzkausener Strasse 14**
**W-4020 Mettmann (DE)**
Erfinder : **Behrenz, Wolfgang, Dr.**
**Untergründemich 14**
**W-5063 Overath (DE)**
Erfinder : **Homeyer, Bernhard, Dr.**
**Obere Strasse 28**
**W-5090 Leverkusen3 (DE)**
Erfinder : **Stendel, Wilhelm, Dr.**
**In den Birken 55**
**W-5600 Wuppertal 1 (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue Saint-Denis, 75001 PARIS

**Beschreibung**

Die Erfindung betrifft neue 1-Arylpyrazole, ein Verfahren zu ihrer Herstellung und ihre Verwendung als Schädlingsbekämpfungsmittel, insbesondere als Insektizide und Akarizide.

Es ist bereits bekannt, daß bestimmte Pyrazol-Derivate, wie beispielsweise das 1-Cyclohexyl-5-[N,N-(dimethyl)-carbamoyloxy]-3-methylthiomethyl-pyrazol, das 1-Cyclohexyl-5-[N,N-(dimethyl)-carbamoyloxy]-3-methylsulfinylmethyl-pyrazol oder das 1-Cyclohexyl-5-[N,N-(dimethyl)-carbamoyloxy]-3-methylsulfonylmethyl-pyrazol insektizide Wirkung besitzen (vgl DE-OS 2 839 270).

Die Wirkungshöhe bzw. Wirkungsdauer dieser Verbindungen sind jedoch, insbesondere bei bestimmten Insekten oder bei niedrigen Anwendungskonzentrationen nicht immer in allen Anwendungsbereichen völlig zufriedenstellend.

Es wurden nun neue 1-Aryl-pyrazole der allgemeinen Formel (I),

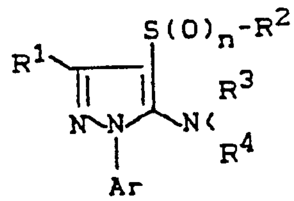

(I)

in welcher

$R^1$ für Wasserstoff, oder für jeweils geradkettiges oder verzweigtes Alkyl oder Halogenalkyl mit 1 bis 4 Kohlenstoffatomen und gegebenenfalls 1 bis 9 gleichen oder verschiedenen Halogenatomen steht,

$R^2$ für jeweils geradkettiges oder verzweigtes Alkyl, Alkenyl oder Alkinyl mit jeweils bis zu 8 Kohlenstoffatomen, für Cycloalkyl mit 3 bis 7 Kohlenstoffatomen, für jeweils geradkettiges oder verzweigtes Halogenalkyl oder Halogenalkenyl mit jeweils bis zu 8 Kohlenstoffatomen und bis zu 17 gleichen oder verschiedenen Halogenatomen, für jeweils geradkettiges oder verzweigtes Alkoxyalkyl, Alkylthioalkyl, Alkylsulfinylalkyl oder Alkylsulfonylalkyl mit jeweils 1 bis 6 Kohlenstoffatomen in den einzelnen Alkylteilen oder für jeweils im Phenylteil gegebenenfalls einfach oder mehrfach, gleich oder verschieden substituiertes Phenylalkyl oder Phenyl mit gegebenenfalls 1 bis 4 Kohlenstoffatomen im geradkettigen oder verzweigten Alkylteil steht, wobei als Substituenten im Phenylteil jeweils infrage kommen : Halogen, Cyano, Nitro, jeweils geradkettiges oder verzweigtes Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen in den einzelnen Alkylteilen und gegebenenfalls 1 bis 9 gleichen oder verschiedenen Halogenatomen,

$R^3$ für jeweils geradkettiges oder verzweigtes Alkyl, Alkenyl, Alkinyl, Halogenalkyl oder Halogenalkenyl mit jeweils bis zu 8 Kohlenstoffatnmen und gegebenenfalls mit 1 bis 17 Halogenatomen steht, außerdem für jeweils geradkettiges oder verzweigtes Alkoxyalkyl oder Alkylthioalkyl mit jeweils 1 bis 4 Kohlenstoffatomen in den einzelnen Alkylteilen, für Cycloalkyl mit 3 bis 7 Kohlenstoffatomen oder für im Phenylteil gegebenenfalls einfach- oder mehrfach, gleich oder verschieden substituiertes, im Alkylteil geradkettiges oder verzweigtes Phenylalkyl mit 1 bis 4 Kohlenstoffatomen im Alkylteil steht, wobei als Phenylsubstituenten die bei $R^2$ genannten infrage kommen,

$R^4$ für jeweils geradkettiges oder verzweigtes Alkyl, Alkenyl, Alkinyl, Halogenalkyl oder Halogenalkenyl mit jeweils bis zu 8 Kohlenstoffatomen und gegebenenfalls mit 1 bis 17 Halogenatomen steht, außerdem für jeweils geradkettiges oder verzweigtes Alkoxyalkyl oder Alkylthioalkyl mit jeweils 1 bis 4 Kohlenstoffatomen in den einzelnen Alkylteilen, für Cycloalkyl mit 3 bis 7 Kohlenstoffatomen oder für im Phenylteil gegebenenfalls einfach- oder mehrfach, gleich oder verschieden substituiertes, im Alkylteilgerad-kettiges oder verzweigtes Phenylalkyl mit 1 bis 4 Kohlenstoffatomen im Alkylteil steht, wobei als Phenylsubstituenten die bei $R^2$ genannten infrage kommen, und darüberhinaus auch für Wasserstoff steht, für den Fall, daß dann $R^3$ nicht gleichzeitig für unsubstituiertes Alkyl steht, oder

$R^3$ und $R^4$ gemeinsam mit dem Stickstoffatom, an welches sie gebunden sind, für einen 3- bis 7- gliedrigen gesättigten Heterocyclus stehen, der bis zu 2 weitere Heteroatome, insbesondere Stickstoff, Sauerstoff und/oder Schwefel enthalten kann,

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten jeweils infrage kommen : Cyano, Nitro, Halogen, jeweils geradkettiges oder verzweigtes Alkyl, Alkoxy oder Alkoxycarbonyl mit jeweils 1 bis 4 Kohlenstoffatomen, jeweils geradkettiges oder verzweigtes Halogenalkyl oder Halogenalkoxy mit jeweils 1 bis 4 Kohlenstoffatomen

und 1 bis 9 gleichen oder verschiedenen Halogenatomen oder ein Rest -$S(O)_p$-$R^6$ und

n für eine Zahl 0, 1 oder 2 steht, wobei

$R^6$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht und

p für eine Zahl 0, 1 oder 2 steht.

Weiterhin wurde gefunden, daß man die neuen 1-Arylpyrazole der allgemeinen Formel (I),

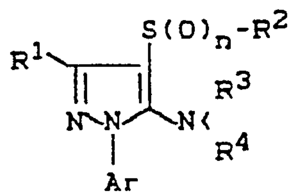

(I)

in welcher

$R^1$, $R^2$, $R^3$, $R^4$, Ar und n die oben angegebene Bedeutung besitzen, mit Hilfe der im folgenden beschriebenen Verfahren erhält:

Man erhält 1-Aryl-pyrazole der Formel (I), wenn man

(a) 5-Halogen-1-aryl-pyrazole der Formel (II),

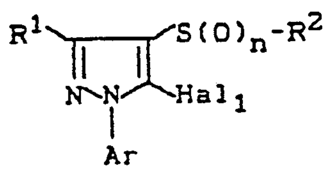

(II)

in welcher

$R^1$, $R^2$, n und Ar die oben angegebene Bedeutung haben und

$Hal^1$ für Halogen steht, mit Aminen der Formel (III),

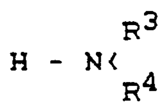

(III)

in welcher

$R^3$ und $R^4$ die oben angegebene Bedeutung haben, gegebenenfalls in Gegenwart eines Verdünnungsmittels und gegebenenfalls in Gegenwart eines Säurebindemittels umsetzt, oder wenn man

(b) 5-Amino-1-aryl-pyrazole der Formel

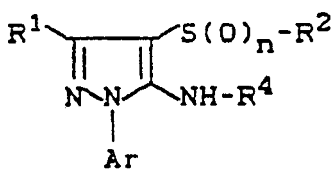

(IV)

in welcher

$R^1$, $R^2$, $R^4$, n und Ar die oben angegebene Bedeutung haben, mit Alkylierungsmitteln der Formel (V),

$R^3$-$E^1$ (V)

in welcher

$R^3$ die oben angegebene Bedeutung hat und

$E^1$ für eine elektronenanziehende Abgangsgruppe steht, gegebenenfalls in Gegenwart eines Verdünnungsmittels, gegebenenfalls in Gegenwart eines Säurebindemittels und gegebenenfalls in Gegenwart eines Katalysators umsetzt ; man erhält 1-Aryl-pyrazole der Formel (Ia),

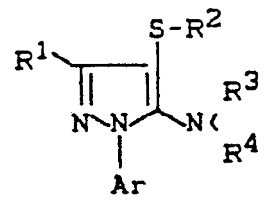

(Ia)

in welcher
$R^1$, $R^2$, $R^3$, $R^4$ und Ar die oben angegebene Bedeutung haben, alternativ auch, wenn man
(c) 4-unsubstituierte 1-Aryl-pyrazole der Formel (VI),

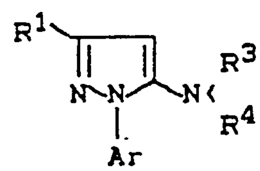

(IV)

in welcher
$R^1$, $R^3$, $R^4$ und Ar die oben angegebene Bedeutung haben, mit Sulfenylhalogeniden der Formel (VII),

$R^2$-S-$Hal^2$ (VII)

in welcher
$R^2$ die oben angegebene Bedeutung hat und
$Hal^2$ für Halogen steht, gegebenenfalls in Gegenwart eines Verdünnungsmittels und gegebenenfalls in Gegenwart eines Säurebindemittels umsetzt ; man erhält 1-Aryl-pyrazole der Formel (Ib),

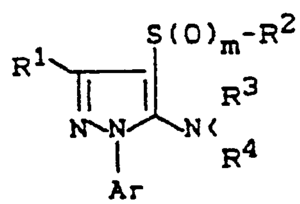

(Ib)

in welcher
$R^1$, $R^2$, $R^3$, $R^4$ und Ar die oben angegebene Bedeutung haben und
m für eine Zahl 1 oder 2 steht,
alternativ auch, wenn man
(d) die nach Verfahren (a), (b) oder (c) erhältlichen 1-Aryl-pyrazole der Formel (Ia),

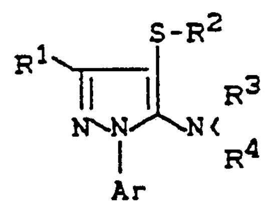

(Ia)

in welcher
$R^1$, $R^2$, $R^3$, $R^4$ und Ar die oben angegebene Bedeutung haben,

mit Oxidationsmitteln der Formel (VIII),

$R^5$-O-O-H (VIII)

in welcher

$R^5$ für Wasserstoff oder für jeweils gegebenenfalls substituiertes Alkanoyl oder Aroyl steht, gegebenenfalls in Gegenwart eines Verdünnungsmittels, gegebenenfalls in Gegenwart eines Katalysators sowie gegebenenfalls in Gegenwart eines Säurebindemittels am Schwefel der Sulfenylgruppe in 4-Position des Pyrazolringes oxidiert.

Schließlich wurde gefunden, daß die neuen 1-Aryl-pyrazole der allgemeinen Formel (I) stark ausgeprägte insektizide und akarizide Eigenschaften besitzen.

Überraschenderweise zeigen die erfindungsgemäßen 1-Aryl-pyrazole der allgemeinen Formel (I) eine erheblich bessere insektizide und akarizide Wirksamkeit, als die aus dem Stand der Technik bekannten Pyrazol-Derivate, wie beispielsweise das 1-Cyclohexyl-5-[N,N-(dimethyl)-carbamoyloxy]-3-methylthiomethyl-pyrazol, das 1-Cyclohexyl-5-[N,N-(dimethyl)-carbamoyloxy]-3-methylsulfinyl-pyrazol oder das 1-Cyclohexyl-5-[N,N-(dimethyl)-carbamoyloxy]-3-methylsulfonylmethyl-pyrazol, welches chemisch und wirkungsmäßig naheliegende Verbindungen sind.

Die erfindungsgemäßen 1-Aryl-pyrazole sind durch die Formel (I) allgemein definiert. Bevorzugt sind Verbindungen der Formel (I), bei welchen

$R^1$ für Wasserstoff, Methyl, Ethyl, n- oder i-Propyl und Trifluormethyl steht,

$R^2$ für Methyl, Ethyl, n- oder i-Propyl, n-, i-, s- oder t-Butyl, n- oder i-Pentyl, n- oder i-Hexyl, Allyl, n- oder i-Butenyl, n- oder i-Pentenyl, Propargyl, n- oder i-Butinyl, Cyclopropyl, Cyclobutyl, Cyclopentyl, Cyclohexyl, Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Chlorpropyl, Brompropyl, Dichlormethyl, Chlorfluormethyl, Trichlormethyl, Dichlormethyl, Trifluorethyl, Trifluorchlorethyl, Tetrafluorethyl, Difluorchlorethyl, Fluordibrommethyl, Difluorbrommethyl, Fluorchlorbrommethyl, Chlorallyl, Fluorallyl, Chlorbutenyl, Fluorbutenyl, Dichlorallyl, Fluorchlorallyl, Dichlorbutenyl, Difluorallyl, Bromallyl, für Methoxymethyl, Methoxyethyl, Methoxypropyl, Ethoxymethyl, Ethoxyethyl, Methylthiomethyl, Methylsulfinylmethyl, Methyl-sulfonylethyl, Methylthioethyl, Methylthiopropyl, Methylsulfinylethyl, Methylsulfonylmethyl oder für jeweils gegebenenfalls einfach bis dreifach, gleich oder verschieden substituiertes Phenyl, Benzyl oder Phenylethyl steht, wobei als Phenylsubstituenten jeweils in Frage kommen : Fluor, Chlor, Brom, Iod, Cyano, Nitro, Methyl, Ethyl, Methoxy, Methylthio, Trifluormethyl, Methylsulfinyl, Methylsulfonyl, Trifluormethoxy, Trifluormethylthio, Trifluormethylsulfinyl oder Trifluormethylsulfonyl,

$R^3$ für Methyl, Ethyl ; n- oder i-Propyl, n-, i-, s- oder t-Butyl, n- oder i-Pentyl, n- oder i-Hexyl, für Allyl, n- oder i-Butenyl, n- oder i-Pentenyl, Propargyl, n- oder i-Butinyl, Cyclopropyl, Cyclobutyl, Cyclopentyl, Cyclohexyl, Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Chlorpropyl, Brompropyl, Dichlormethyl, Chlorfluormethyl, Trichlormethyl, Trifluorethyl, Trifluorchlorethyl, Tetrafluorethyl, Difluorchlorethyl, Fluordibrommethyl, Difluorbrommethyl, Fluorchlorbrommethyl, Chlorallyl, Fluorallyl, Chlorbutenyl, Fluorbutenyl, Dichlorallyl, Fluorchlorallyl, Dichlorbutenyl, Difluorallyl, Bromallyl, für Methoxymethyl, Methoxyethyl, Methoxypropyl, Ethoxymethyl, Ethoxyethyl, Methylthiomethyl, Methylthioethyl, Methylthiopropyl, Ethylthiomethyl, Ethylthioethyl oder für jeweils im Phenylteil gegebenenfalls ein- bis dreifach, gleich oder verschieden substituiertes Benzyl oder Phenylethyl steht, wobei als Phenylsubstituenten die bei $R^2$ genannten infrage kommen,

$R^4$ für Methyl, Ethyl, n- oder i-Propyl, n-, i-, s- oder t-Butyl, n- oder i-Pentyl, n- oder i-Hexyl, für Allyl, n- oder i-Butenyl, n- oder i-Pentenyl, Propargyl, n- oder i-Butinyl, Cyclopropyl, Cyclobutyl, Cyclopentyl, Cyclohexyl, Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Chlorpropyl, Brompropyl, Dichlormethyl, Chlorfluormethyl, Trichlormethyl, Trifluorethyl, Trifluorchlorethyl, Tetrafluorethyl, Difluorchlorethyl, Fluordibrommethyl, Difluorbrommethyl, Fluorchlorbrommethyl, Chlorallyl, Fluorallyl, Chlorbutenyl, Fluorbutenyl, Dichlorallyl, Fluorchlorallyl, Dichlorbutenyl, Difluorallyl, Bromallyl, für Methoxymethyl, Methoxyethyl, Methoxypropyl, Ethoxymethyl, Ethoxyethyl, Methylthiomethyl, Methylthioethyl, Methylthiopropyl, Ethylthiomethyl, Ethylthioethyl oder für jeweils im Phenylteil gegebenenfalls ein- bis dreifach, gleich oder verschieden substituiertes Benzyl oder Phenylethyl steht, wobei als Phenylsubstituenten die bei $R^2$ genannten infrage kommen, und darüberhinaus auch für Wasserstoff steht, für den Fall, daß dann $R^3$ nicht gleichzeitig für unsubstituiertes Alkyl steht, oder

$R^3$ und $R^4$ gemeinsam mit dem Stickstoffatom, an welches sie gebunden sind, für Pyrrolidinyl, Piperidinyl,

Perhydroazepinyl oder Morpholinyl stehen,

Ar für gegebenenfalls ein- bis fünffach, gleich oder verschieden substituiertes Phenyl oder für gegebenenfalls ein- bis vierfach, gleich oder verschieden substituiertes 2-Pyridyl steht, wobei als Substituenten jeweils infrage kommen : Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_p-R^6$ und

n für eine Zahl 0, 1 oder 2 steht, wobei

$R^6$ für Amino, Methylamino, Ethylamino, Dimethylamino, Diethylamino, Fluordichlormethyl, Difluormethyl, Tetrafluorethyl, Trifluorchlorethyl, Trichlormethyl, Trichlorethyl, Trifluormethyl, Methyl oder Ethyl steht und

p für eine Zahl 0, 1 oder 2 steht

Im einzelnen seien außer den bei den Herstellungsbeispielen genannten Verbindungen die folgenden 1-Aryl-pyrazole der allgemeinen Formel (I) genannt :

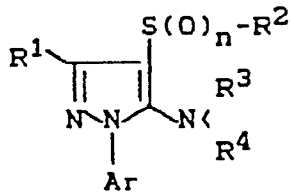

(I)

| $R^1$ | $-S(O)_n-R^2$ | $-N\langle^{R^3}_{R^4}$ | Ar |
|---|---|---|---|
| $CH_3$ | $-SCF_3$ | $-N\langle^{CH_3}_{CH_3}$ | 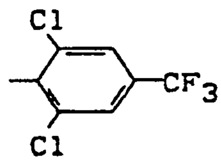 |
| $CH_3$ | $-SO_2CF_3$ | $-N\langle^{C_2H_5}_{C_2H_5}$ | 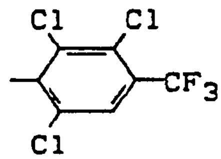 |
| $CH_3$ | $-SCCl_2F$ | $-N\langle^{CH_3}_{CH_3}$ | 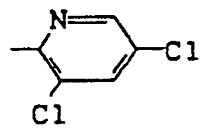 |
| H | $-SCCl_2F$ | $-N\langle^{CH_3}_{CH_3}$ | 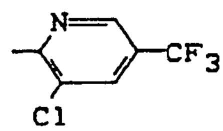 |
| H | $-SCCl_2F$ | $-N\langle^{CH_3}_{CH_3}$ | 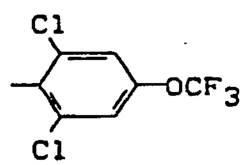 |
| H | $-SO-CCl_2F$ | $-N\langle^{C_2H_5}_{C_2H_5}$ | 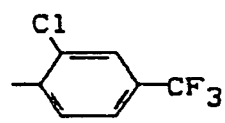 |

| $R^1$ | $-S(O)_n-R^2$ | $-N<^{R^3}_{R^4}$ | Ar |
|---|---|---|---|
| $CH_3$ | $-SO_2-CCl_2F$ | $-N<^{CH_3}_{C_2H_5}$ | 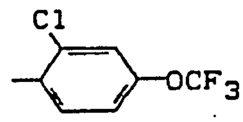 |
| $C_2H_5$ | $-S-CF_3$ | 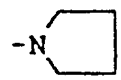 | 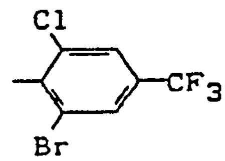 |
| H | $-SCF_3$ | 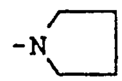 | 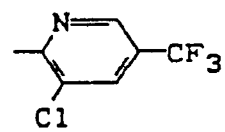 |
| H | $-S-CH_3$ | 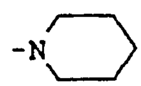 | 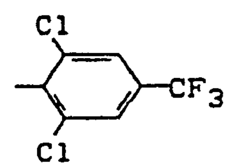 |
| $CH_3$ | $-S-CH_3$ | 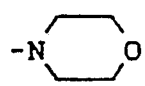 | 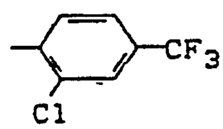 |
| $CH_3$ | $-SO_2-CH_3$ | 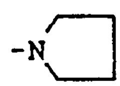 | 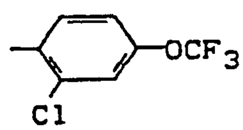 |
| $CH_3$ | $-SO_2-C_2H_5$ | $-N<^{CH_3}_{CH_3}$ | 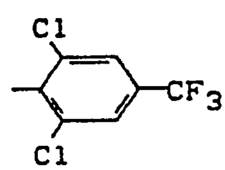 |
| H | $-S-CClF_2$ | $-NH-CH_2-CH_2OCH_3$ | 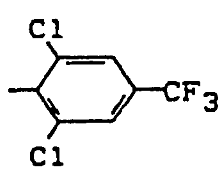 |

| $R^1$ | $-S(O)_n-R^2$ | $-N\langle^{R^3}_{R^4}$ | Ar |
|---|---|---|---|
| $CH_3$ | $-SO_2-CClF_2$ | $-NH-CH_2-SCH_3$ | 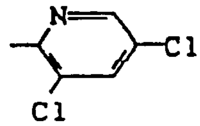 |
| H | $-SO_2CF_3$ | $-N\langle^{CH_3}_{CH_3}$ | 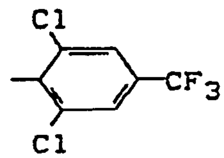 |
| H | $-SCF_3$ | $-N\langle^{CH_3}_{CH_3}$ | 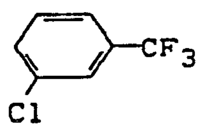 |
| H | $-SCF_3$ | $-N\langle^{CH_3}_{CH_3}$ | 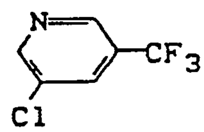 |
| $CH_3$ | $-SO_2CF_3$ | $-N\langle^{CH_3}_{CH_3}$ | 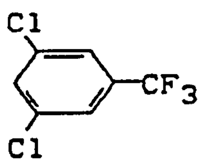 |
| H | $-SO_2CF_3$ | 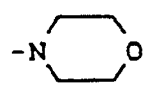 | 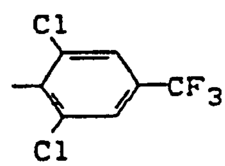 |
| H | $-SO_2CF_3$ | $-NH-CH_2-CH_2-OCH_3$ | 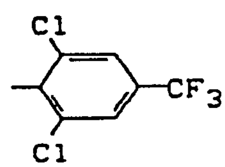 |
| H | $-SO_2CF_3$ | $-N\langle^{CH_2-CH_2-OCH_3}_{CH_2-CH_2-OCH_3}$ | 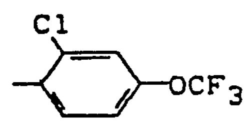 |

| $R^1$ | $-S(O)_n-R^2$ | $-N<^{R^3}_{R^4}$ | Ar |
|---|---|---|---|
| H | $-SOCF_3$ | $-N<^{C_2H_5}_{C_2H_5}$ | 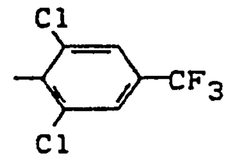 |
| H | $-SO_2CF_3$ | $-N<^{CH_3}_{C_2H_5}$ | 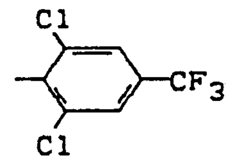 |

Verwendet man beispielsweise 5-Brom-1-(2,6-dichlor-4-trifluormethyl-phenyl)-4-fluordichlormethylsulfonyl-pyrazol und 2-Methoxyethylamin als Ausgangsstoffe, läßt sich der Reaktionsablauf des erfindungsgemäßen Verfahrens (a) durch das folgende Formelschema darstellen :

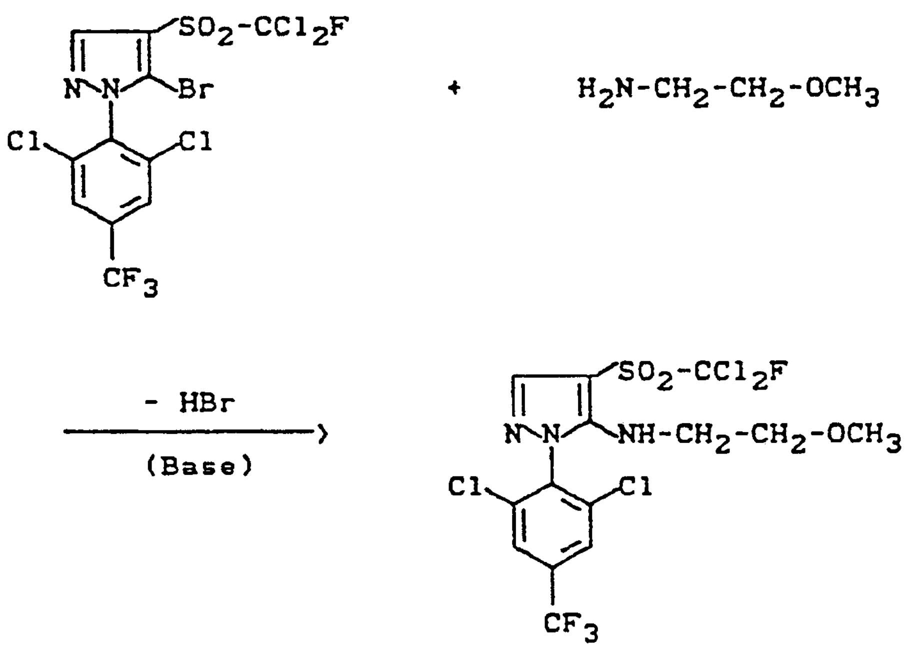


Verwendet man beispielsweise 5-Amino-1-(2,6-dichlor-4-trifluormethyl-phenyl)-4-fluordichlormethylthiopyrazol und Dimethylsulfat als Ausgangsstoffe, so läßt sich der Reaktionsablauf des erfindungsgemäßen Verfahrens (b) durch das folgende Formelschema darstellen :

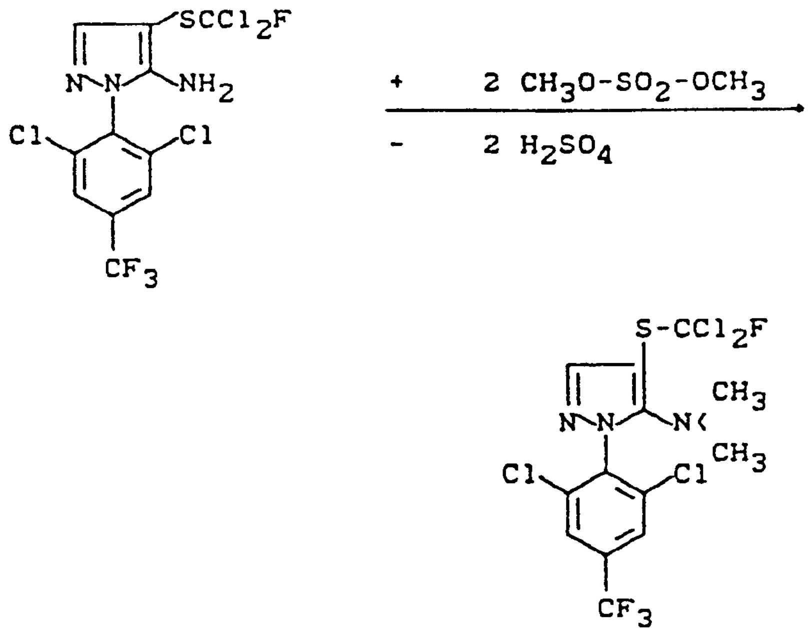


Verwendet man beispielsweise 3-Methyl-5-dimethylamino-1-(2-chlor-4-trifluormethyl-phenyl)-pyrazol und Trifluormethylsulfenylchlorid als Ausgangsstoffe, so läßt sich der Reaktionsablauf des erfindungsgemäßen Verfahrens (c) durch das folgende Formelschema darstellen :

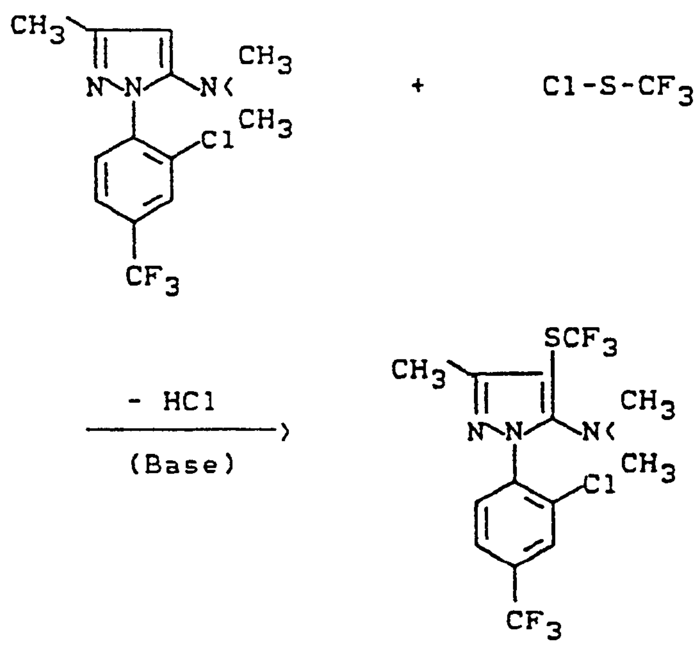


Verwendet man beispielsweise 5-Diethylamino-3-methyl-4-trifluormethylthio-1-(2,4,6-trichlorphenyl)-pyrazol als Ausgangsstoff und 3-Chlorperbenzoesäure als Oxidationsmittel, so läßt sich der Reaktionsablauf des erfindungsgemäßen Verfahrens (d) durch das folgende Formelschema darstellen :

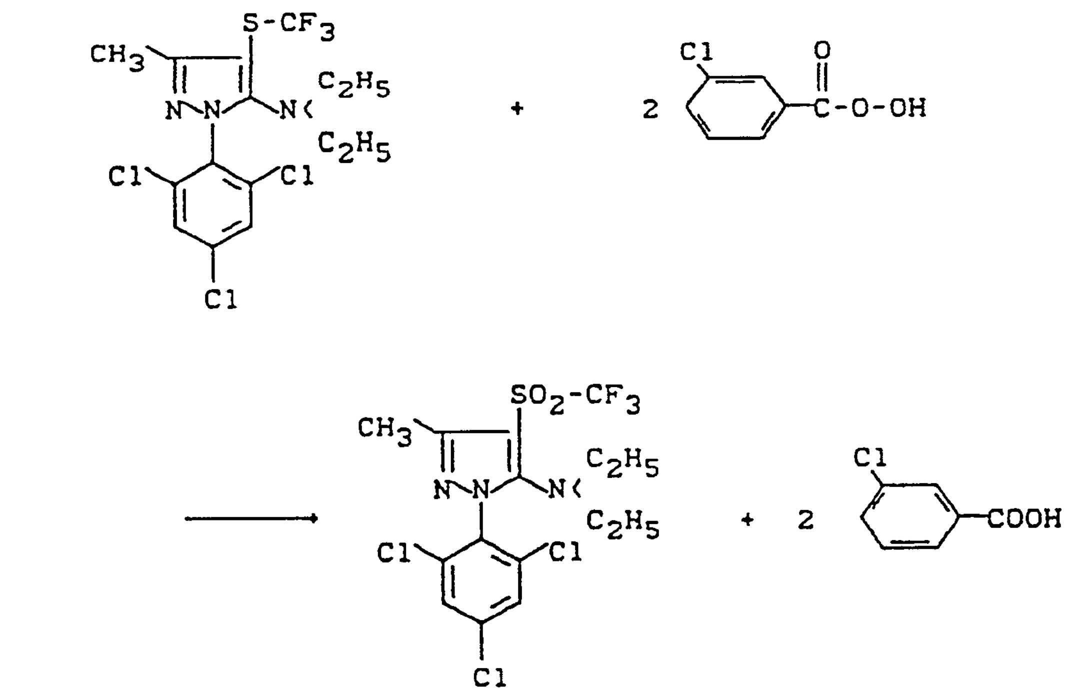

Die zur Durchführung des erfindungsgemäßen Verfahrens (a) als Ausgangsstoffe benötigten 5-Halogen-1-aryl-pyrazole sind durch die Formel (II) allgemein definiert. In dieser Formel (II) stehen $R^1$, $R^2$, Ar und n vorzugsweise für diejenigen Reste und Indices, die bereits im Zusammenhang mit der Beschreibung der erfindungsgemäßen Stoffe der Formel (I) als bevorzugt für diese Indices und Substituenten genannt wurden.

$Hal^1$ steht vorzugsweise für Chlor oder Brom.

Die 5-Halogen-1-aryl-pyrazole der Formel (II) sind noch nicht bekannt. Sie sind jedoch größtenteils Gegenstand der eigenen vorgängigen noch nicht publizierten Patentanmeldung DE-P 3 529 829 vom 21.08.1985 (LeA 23917).

Man erhält sie beispielsweise, wenn man 5-Amino-1-aryl-pyrazole der Formel (IVa),

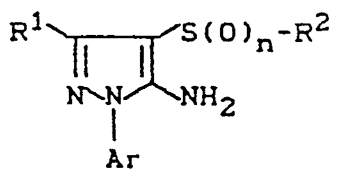

(IVa)

in welcher

$R^1$, $R^2$, Ar und n die oben angegebene Bedeutung haben,

mit einem anorganischen oder organischen Nitrit, wie beispielsweise Natriumnitrit oder t-Butylnitrit in Gegenwart eines Halogenierungsmittels wie beispielsweise Chlorwasserstoffsäure oder Bromoform bei Temperaturen zwischen 0°C und + 80°C umsetzt.

Die zur Durchführung des erfindungsgemäßen Verfahrens (a) und der Synthese der Vorprodukte der Formel (VIb) weiterhin als Ausgangsstoffe benötigten Amine sind durch die Formel (III) allgemein definiert. In dieser Formel (III) stehen $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden.

Die Amine der Formel (III) sind allgemein bekannte Verbindungen der organischen Chemie.

Die zur Durchführung des erfindungsgemäßen Verfahrens (b) als Ausgangsstoffe benötigten 5-Amino-1-aryl-pyrazole sind durch die Formel (IV) allgemein definiert. In dieser Formel (IV) stehen $R^1$, $R^2$, $R^4$, Ar und n vorzugsweise für diejenigen Reste und Indices, die bereits im Zusammenhang mit der Beschreibung der erfindungsgemäßen Stoffe der Formel (I) als bevorzugt für diese Indices und Substituenten genannt wurden.

Die 5-Amino-1-aryl-pyrazole der Formel (IV) ebenso wie die zur Herstellung der Vorprodukte der Formel (II) benötigten 5-Amino-1-aryl-pyrazole der Formel (IVa) sind teilweise bekannt (vgl. z.B. Farmaco. Ed. Sci. 26, S. 276-293 [1971], DE-OS 3 402 308, oder Mycopathologica 74, S. 7-14 [1981], teilweise sind sie Gegenstand

der eigenen noch nicht veröffentlichten vorgängigen Patentanmeldung DE-P 3 517 843 vom 16.05.1985 [LeA 23753] und teilweise sind sie erfindungsgemäße Verbindungen und erhältlich mit Hilfe der erfindungsgemäßen Verfahren (a), (b), (c) oder (d).

Man erhält die vorbeschriebenen 5-Amino-1-aryl-pyrazole der Formel (IV) bzw. (IVa) in Analogie zu bekannten Verfahren, beispielsweise, wenn man 4-Thiocyanato-5-aminopyrazole der allgemeinen Formel (IX),

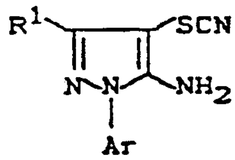

(IX)

in welcher

$R^1$ und Ar die oben angegebene Bedeutung haben, oder Bis-(pyrazolyl)-disulfide der Formel (X),

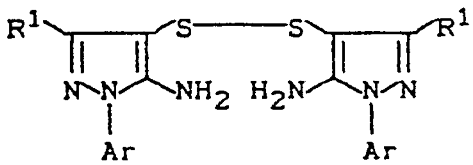

(X)

in welcher

$R^1$ und Ar die oben angegebene Bedeutung haben, mit Halogeniden der Formel (XI)

$R^2$-$Hal^3$ (XI)

in welcher

$R^2$ die oben angegebene Bedeutung hat und

$Hal^3$ für Halogen, insbesondere für Chlor, Brom oder Iod steht, gegebenenfalls in Gegenwart eines Verdünnungsmittels wie beispielsweise Methanol oder Ethanol sowie gegebenenfalls in Gegenwart eines Reduktionsmittels wie beispielsweise Natriumborhydrid oder Natriumdithionit und gegebenenfalls in Gegenwart einer Base, wie beispielsweise Natriumhydroxid oder Kaliumcarbonat bei Temperaturen zwischen 20°C und 90°C umsetzt, oder wenn man 4-unsubstituierte 5-Amino-pyrazole der Formel (VIa),

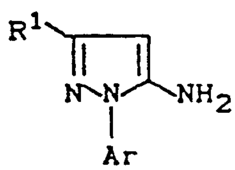

(VIa)

in welcher

$R^1$ und Ar die oben angegebene Bedeutung haben, mit Sulfenylhalogeniden der Formel (VII),

$R^2$-S-$Hal^2$ (VII)

in welcher

$R^2$ die oben angegebene Bedeutung hat und

$Hal^2$ für Halogen, insbesondere für Fluor, Chlor, Brom oder Iod steht, in Analogie zur Durchführung des erfindungsgemäßen Verfahrens (c) gegebenenfalls in Gegenwart eines Verdünnungsmittels wie beispielsweise Dichlormethan und gegebenenfalls in Gegenwart eines Säurebindemittels wie beispielsweise Pyridin bei Temperaturen zwischen 0°C und 50°C umsetzt, und gegebenenfalls anschließend die so erhältlichen 5-Amino-pyrazole der Formel (IVb),

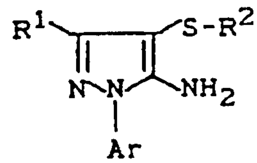

(IVb)

in welcher

$R^1$, $R^2$ und Ar die oben angegebene Bedeutung haben, mit Oxidationsmitteln der Formel (VIII),

$R^5$-O-OH (VIII)

in welcher

$R^5$ für Wasserstoff oder für jeweils gegebenenfalls substituiertes Alkanoyl oder Aroyl, vorzugsweise für Wasserstoff, für Acetyl, für Propionyl, für Trifluoracetyl oder für gegebenenfalls substituiertes Benzoyl wie beispielsweise 3-Chlorbenzoyl oder 4-Nitrobenzoyl steht, in Analogie zur Durchführung des erfindungsgemäßen Verfahrens (d) gegebenenfalls in Gegenwart eines Verdünnungsmittels wie beispielsweise Dichlormethan, gegebenenfalls in Gegenwart eines Katalysators wie beispielsweise Ammoniummolybdat sowie gegebenenfalls in Gegenwart eines Säurebindemittels wie beispielsweise Natriumcarbonat oder Natriumbicarbonat bei Temperaturen zwischen 0°C und 50°C am Schwefel der Sulfenylgruppe in 4-Position des Pyrazolringes oxidiert. 5-Amino-1-aryl-pyrazole der Formel (IVd),

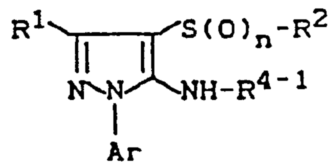

(IVd)

in welcher

$R^1$, $R^2$, Ar und n die oben angegebene Bedeutung haben und

$R^{4-1}$ für die gleichen Reste wie $R^4$ mit Ausnahme des Wasserstoffrestes steht, erhält man entweder durch Umsetzen der Vorprodukte der Formel (II),

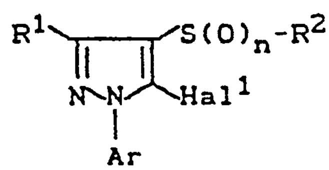

(II)

in welcher

$R^1$, $R^2$, Ar, $Hal^1$ und n die oben angegebene Bedeutung haben, mit Aminen der Formel (IIIa),

$H_2N-R^{4-1}$ (IIIA)

in welcher

$R^{4-1}$ die oben angegebene Bedeutung hat, in Analogie zur Durchführung des erfindungsgemäßen Verfahrens (a), oder durch Umsetzen der Vorprodukte der Formel (IVa),

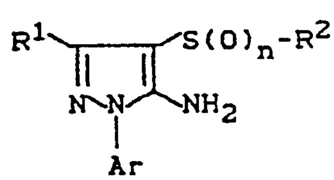

(IVa)

in welcher

$R^1$, $R^2$, Ar und n die oben angegebene Bedeutung haben, mit Alkylierungsmitteln der Formel (Va),

$$R^{4-1}\text{-}E^1 \quad \text{(VA)}$$

in welcher

$R^{4-1}$ und $E^1$ die oben angegebene Bedeutung haben, in Analogie zur Durchführung des erfindungsgemäßen Verfahrens (b).

Die Amine der Formel (IIIa) und die Alkylierungsmittel der Formel (Va) sind allgemein bekannte Verbindungen der organischen Chemie.

Die 4-Thiocyanato-5-aminopyrazole der Formel (IX) sind teilweise bekannt (vgl. z.B. Farmaco Ed. Sci. 38, S. 274-282 [1983]). Man erhält sie beispielsweise, wenn man 4-unsubstituierte 5-Aminopyrazole der Formel (VIa),

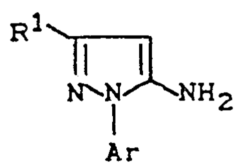

(VIa)

in welcher

$R^1$ und Ar die oben angegebene Bedeutung haben, mit Ammoniumthiocyanat in Gegenwart von Brom und Essigsäure bei Temperaturen zwischen –20°C und +20°C umsetzt.

Die Bis-(pyrazol)-disulfide der Formel (X) sind noch nicht bekannt. Man erhält sie, wenn man die oben beschriebenen 4-Thiocyanato-5-amino-pyrazole der Formel (IX) mit wässriger Salzsäure, gegebenenfalls in Gegenwart eines Verdünnungsmittels, wie beispielsweise Ethanol bei Temperaturen zwischen 20°C und 120°C umsetzt.

Die Halogeniden der Formel (XI) sind allgemein bekannte Verbindungen der organischen Chemie.

Die 4-unsubstituierten 5-Aminopyrazole der Formel (VIa) sind teilweise bekannt (vgl. z.B. J. Org. Chem. 36, S. 2972-2974 [1971] oder J. Heterocyclic Chemistry 7, S. 345-349 [1970] ; C.A. 62 : 13137c).

Man erhält sie beispielsweise, wenn man Arylhydrazine der Formel (XII),

$$Ar\text{-}NH\text{-}NH_2 \quad \text{(XII)}$$

in welcher

Ar die oben angegebene Bedeutung hat,

mit Acrylnitrilderivaten der Formel (XIII),

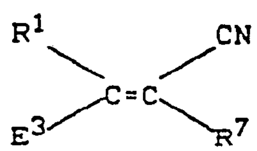

(XIII)

in welcher

$R^1$ die oben angegebene Bedeutung hat,

$R^7$ für Wasserstoff oder Alkoxycarbonyl steht und

$E^3$ für Halogen, Hydroxy, Alkoxy, Amino oder Dialkylamino steht, entweder zunächst in einer 1. Stufe, gegebenenfalls in Gegenwart eines Verdünnungsmittels wie beispielsweise Ethanol oder Eisessig und gegebenenfalls in Gegenwart eines Reaktionshilfsmittels wie beispielsweise Natriumacetat bei Temperaturen zwischen –20°C und +20°C umsetzt zu den Arylhydrazinderivaten der Formel (XIV),

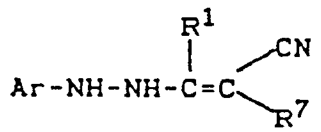

(XIV)

in welcher

Ar, $R^1$ und $R^7$ die oben angegebene Bedeutung haben,

und diese in einer 2. Stufe, gegebenenfalls in Gegenwart eines Verdünnungsmittels wie beispielsweise Ethylenglykolmonoethylether bei Temperaturen zwischen +50°C und +150°C cyclisiert, oder direkt in einem Reaktionsschritt ohne Isolierung der Zwischenstufe der Formel (XIV), gegebenenfalls in Gegenwart eines Verdünnungsmittels wie beispielsweise Ethylenglykolmonoethylether bei Temperaturen zwischen +50°C und +150°C cyclisiert zu den 5-Amino-pyrazolen der Formel (XV),

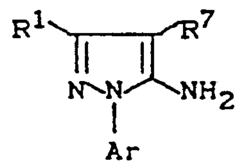

(XV)

in welcher

$R^1$, $R^7$ und Ar die oben angegebene Bedeutung haben, und in dem Fall, wo $R^7$ für Alkoxycarbonyl steht, die Verbindungen der Formel (XV), in allgemein üblicher Art und Weise, gegebenenfalls in Gegenwart eines Verdünnungsmittels, wie beispielsweise Ethanol oder Isopropanol sowie gegebenenfalls in Gegenwart eines Katalysators wie beispielsweise Bromwasserstoffsäure, bei Temperaturen zwischen 50°C und 150°C verseift und decarboxyliert.

Die Arylhydrazine der Formel (XII) sind bekannt (vgl. z.B. US-PS 4 127 575 ; US-PS 3 609 158 ; DE-OS 2 558 399 ; J. Chem. Soc. C, 1971, S. 167-174) oder lassen sich nach prinzipiell bekannten Verfahren in einfacher analoger Weise herstellen (vgl. Houben-Weyl "Methoden der organischen Chemie" Band X, 2 S. 203, Thieme Verlag Stuttgart 1967).

Die Acrylnitrilderivate der Formel (XIII) sind allgemein bekannte Verbindungen der organischen Chemie.

Die zur Durchführung des erfindungsgemäßen Verfahrens (b) und zur Synthese der Vorprodukte der Formel (VIb) weiterhin als Ausgangsstoffe benötigten Alkylierungsmittel sind durch die Formel (V) allgemein definiert. In dieser Formel (V) steht $R^3$ vorzugsweise für diejenigen Reste, die bereits im Zusammenhang mit der Beschreibung der erfindungsgemäßen Stoffe der Formel (I) als bevorzugt für diesen Substituenten genannt wurden.

$E^1$ steht vorzugsweise für Halogen, insbesondere für Chlor, Brom oder Iod oder für jeweils gegebenenfalls substituiertes Alkylsulfonyloxy, Alkoxysulfonyloxy oder Arylsulfonyloxy, wie beispielsweise Methoxysulfonyloxy oder p-Tolylsulfonyloxy.

Die Alkylierungsmittel der Formel (V) sind allgemein bekannte Verbindungen der organischen Chemie.

Die zur Durchführung des erfindungsgemäßen Verfahrens (c) als Ausgangsstoffe benötigten 4-unsubstituierten 1-Aryl-pyrazole sind durch die Formel (VI) allgemein definiert. In dieser Formel (VI) stehen $R^1$, $R^3$, $R^4$ und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die 4-unsubstituierten 1-Aryl-pyrazole der Formel (VI) sind teilweise bekannt (vgl. z.B. Heterocycles 4, S. 1921-1932, [1976] ; Bull. Soc. Chim. France 1970, S. 4436-4438 ; Gazz. Chim. Ital. 97, S. 410-420 [1967] ; DDR-Patent 208466 vom 02.05.1984 bzw. CA 101, 230514d].

Noch nicht bekannt sind 4-unsubstituierte 1-Aryl-pyrazole der Formel (VIb),

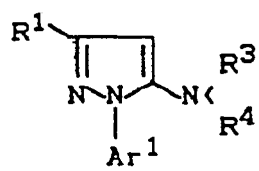

(VIb)

in welcher

$R^1$, $R^3$ und $R^4$ die oben angegebene Bedeutung haben und

$Ar^1$ für zweifach substituiertes Phenyl oder für gegebenenfalls substituiertes Pyridyl steht.

Man erhält sie [ebenso wie die bekannten Verbindungen der Formel (VI) in Analogie zu bekannten Verfahren], beispielsweise, wenn man 4-unsubstituierte 1-Aryl-pyrazole der Formel ($VIa^1$),

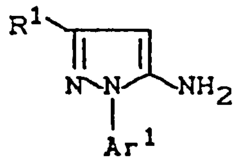

($VIa^1$)

in welcher

$R^1$ und $Ar^1$ die oben angegebene Bedeutung-haben, mit Alkylierungsmitteln der Formel (V),

$$R^3\text{-}E^1 \quad (V)$$

in welcher

$R^3$ und $E^1$ die oben angegebene Bedeutung haben, in Analogie zur Durchführung des erfindungsgemäßen Verfahrens (b) gegebenenfalls in Gegenwart eines Verdünnungsmittels wie beispielsweise Acetonitril und gegebenenfalls in Gegenwart eines Säurebindemittels, wie beispielsweise Kaliumcarbonat bei Temperaturen zwischen 0°C und 120°C alkyliert, oder wenn man 5-Halogen-1-aryl-pyrazole der Formel (XVI),

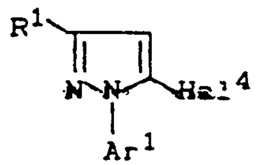

(XVI)

in welcher

$R^1$ und $Ar^1$ die oben angegebene Bedeutung haben und

$Hal^4$ für Halogen, insbesondere für Chlor oder Brom steht, mit Aminen der Formel (III),

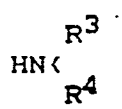

(III)

in welcher

$R^3$ und $R^4$ die oben angegebene Bedeutung haben, in Analogie zur Durchführung des erfindungsgemäßen Verfahrens (a) gegebenenfalls in Gegenwart eines Verdünnungsmittels wie beispielsweise Dioxan und gegebenenfalls in Gegenwart eines Säurebindemittels wie beispielsweise Triethylamin bei Temperaturen zwischen +20°C und +120°C umsetzt.

Die 5-Halogen-1-aryl-pyrazole der Formel (XVI) sind bekannt (vgl. z.B. Bull. Soc. Chim. France 1966, S. 3744-52 ; Canad. J. Chem. 46, S. 1079-1091 [1968] ; Bull. Soc. Chim. France 1968, S. 5019-5029), oder können nach bekannten Verfahren in einfacher, analoger Weise erhalten werden (vgl. z.B. Khim Geterotsikl. Soedin. 1967, S. 130-134 ; Tetrahedron Letters 1968, S. 1949-1951 ; J. Heterocycl. Chem. 18, S. 9-14 [1981]).

Die zur Durchführung des erfindungsgemäßen Verfahrens (c) und zur Synthese der Vorprodukte der Formel (IVb) weiterhin als Ausgangsstoffe benötigten Sulfenylhalogenide sind durch die Formel (VII) allgemein definiert. In dieser Formel (VII) steht $R^2$ vorzugsweise für diejenigen Reste, die bereits im Zusammenhang mit der Beschreibung der erfindungsgemäßen Stoffe der Formel (I) als bevorzugt für diesen Substituenten genannt wurden.

$Hal^2$ steht vorzugsweise für Fluor, Chlor, Brom oder Iod.

Die Sulfenylhalogenide der Formel (VII) sind allgemein bekannte Verbindungen der organischen Chemie.

Die zur Durchführung des erfindungsgemäßen Verfahrens (d) als Ausgangsstoffe benötigten 1-Aryl-pyrazole sind durch die Formel (Ia) allgemein definiert. In dieser Formel (Ia) stehen $R^1$, $R^2$, $R^3$, $R^4$ und Ar vorzugs-

weise für diejenigen Reste, die bereits im Zusammenhang mit der Beschreibung der erfindungsgemäßen Stoffe der Formel (I) als bevorzugt für diese Substituenten genannt wurden.

Die 1-Aryl-pyrazole der Formel (Ia) sind erfindungsgemäße Verbindungen und erhältlich mit Hilfe der erfindungsgemässen Verfahren (a), (b) oder (c).

Die zur Durchführung des erfindungsgemäßen Verfahrens (d) und zur Synthese der Vorprodukte der Formel (IV) weiterhin als Ausgangsstoffe benötigten Oxidationsmittel sind durch die Formel (VIII) allgemein definiert. In dieser Formel (VIII) steht $R^5$ vorzugsweise für Wasserstoff, für Acetyl oder für gegebenenfalls substituiertes Benzoyl, wie beispielsweise 3-Chlorbenzoyl oder 4-Nitrobenzoyl.

Die Oxidationsmittel der Formel (VIII) sind ebenfalls allgemein bekannte Verbindungen der organischen Chemie.

Als Verdünnungsmittel zur Durchführung des Herstellungsverfahrens (a) kommen inerte organische Lösungsmittel infrage. Hierzu gehören insbesondere aliphatische alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (a) kann gegebenenfalls in Gegenwart eines geeigneten Säurebindemittels durchgeführt werden. Als solche kommen alle üblichen anorganischen oder organischen Basen in Frage. Hierzu gehören beispielsweise Alkalimetallhydroxide, wie Natriumhydroxdid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Es ist jedoch auch möglich, einen entsprechenden Überschuß an dem als Reaktionspartner eingesetzten Amin der Formel (III) gleichzeitig als Säurebindemittel zu verwenden.

Die Reaktionstemperaturen können bei der Durchführung des erfindungsgemäßen Verfahrens (a) in einem größeren Bereich variiert werden. Im allgemeinen arbeitet man bei Temperaturen zwischen –20°C und +200°C, vorzugsweise bei Temperaturen zwischen 0°C und +150°C

Zur Durchführung des erfindungsgemäßen Verfahrens (a) setzt man pro Mol an 5-Halogen-1-aryl-pyrazol der Formel (II) im allgemeinen 1.0 bis 10.0 Mol, vorzugsweise 1.0 bis 5.0 Mol an Amin der Formel (III) ein. Die Reaktionsdurchführung, Aufarbeitung und Isolierung der Reaktionsprodukte der Formel (I) erfolgt nach allgemein üblichen Verfahren.

Als Verdünnungsmittel zur Durchführung des erfindungsgemäßen Verfahrens b) kommen inerte organische Lösungsmittel infrage. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (b) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}/C_{15}$-alkylammoniumchlorid, Dibenzyl-ammoniummethylsulfat, Dimethyl-$C_{12}/C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid.

Als Säurebindemittel zur Durchführung des Herstellungsverfahrens (b) kommen alle üblicherweise verwendbaren anorganischen und organischen Basen infrage. Vorzugsweise verwendet man Alkalimetallhydride, -hydroxide, -amide, -alkoholate, -carbonate oder hydrogencarbonate, wie beispielsweise Natriumhydrid, Natriumamid, Natriummethylat, Natriumhydroxid, Natriumcarbonat oder Natriumhydrogencarbonat oder auch tertiäre Amine, wie beispielsweise Triethylamin, N,N-Dimethylanilin, Pyridin, 4-(N,N-Dimethylamino) pyridin, Diazabicyclooctan (DABCO), Diazabicyclononen (DBN) oder Diazabicycloundecen (DBU).

Die Reaktionstemperaturen können bei der Durchführung des Herstellungsverfahrens (b) in einem größeren Bereich variiert werden. Im allgemeinen arbeitet man zwischen –20°C und +150°C, vorzugsweise zwischen 0°C und +100°C.

Zur Durchführung des Herstellungsverfahrens (b) setzt man pro Mol 5-Amino-1-aryl-pyrazol der Formel (IV)

im allgemeinen 1.0 bis 20.0 Mol, vorzugsweise 1.0 bis 15.0 Mol an Alkylierungsmittel der Formel (V) und gegebenenfalls 1.0 bis 3.0 Mol, vorzugsweise 1.0 bis 2.0 Mol an Säurebindemittel sowie 0.01 bis 1.0 Mol an Phasentransferkatalysator ein. Die Reaktionsdurchführung, Aufarbeitung und Isolierung der Reaktionsprodukte der Formel (I) erfolgt in allgemein üblicher Art und Weise.

Als Verdünungsmittel zur Durchführung des erfindungsgemässen Verfahrens (c) kommen inerte organische Lösungsmittel in Frage. Hierzu gehören insbesondere aliphatische oder aromatische, gegebenenfalls halogenierte Kohlenwasserstoffe, wie'beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (c) wird gegebenenfalls in Gegenwart eines Säurebindemittels durchgeführt Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen(DBU).

Die Reaktionstemperaturen können bei der Durchführung des erfindungsgemäßen Verfahrens (c) in einem größeren Bereich variiert werden. Im allgemeinen arbeitet man bei Temperaturen zwischen −20°C und +120°C, vorzugsweise bei Temperaturen zwischen 0°C und +50°C.

Zur Durchführung des erfindungsgemäßen Verfahrens (c) setzt man pro Mol an 4-unsubstituierten 1-Arylpyrazol der Formel (VI) im allgemeinen 1.0 bis 2.5 Mol, vorzugsweise 1.0 bis 1.5 Mol an Sulfenylhalogenid der Formel (VII) und 1.0 bis 2.5 Mol, vorzugsweise 1.0 bis 1.5 Mol an Säurebindemittel ein Die Reaktionsführung, Aufarbeitung und Isolierung der Reaktionsprodukte der Formel (Ia) erfolgt nach allgemein üblichen Verfahren.

Als Verdünnungsmittel zur Durchführung des erfindungsgemäßen Verfahrens (d) kommen ebenfalls inerte organische Lösungsmittel in Frage.

Vorzugsweise verwendet man Kohlenwasserstoffe, wie Benzin, Benzol, Toluol, Hexan oder Petrolether ; chlorierte Kohlenwasserstoffe, wie Dichlormethan, 1,2-Dichlorethan, Chloroform, Tetrachlorkohlenstoff oder Chlorbenzol ; Ether, wie Diethylether, Dioxan oder Tetrahydrofuran ; Carbonsäuren, wie Essigsäure oder Propionsäure, oder dipolar aprotische Lösungsmittel, wie Acetonitril, Aceton, Essigsäureethylester oder Dimethylformamid.

Das erfindungsgemäße Verfahren (d) kann gegebenenfalls in Gegenwart eines Säurebindemittels durchgeführt werden Als solche kommen alle üblicherweise verwendbaren organischen und anorganischen Säurebindemittel in Frage. Vorzugsweise verwendet man Erdalkali- oder Alkalimetallhydroxide, -acetate oder -carbonate, wie beispielsweise Calciumhydroxid, Natriumhydroxid, Natriumacetat oder Natriumcarbonat.

Das erfindungsgemäße Verfahren (d) kann gegebenenfalls in Gegenwart eines geeigneten Katalysators durchgeführt werden. Als solche kommen alle üblicherweise für derartige Schwefeloxidationen üblichen Katalysatoren in Frage. Beispielhaft genannt seien in diesem Zusammenhang Schwermetallkatalysatoren wie Ammoniummolybdat.

Die Reaktionstemperaturen können bei der Durchführung des erfindungsgemäßen Verfahrens (d) in einem größeren Bereich variiert werden Im allgemeinen arbeitet man bei Temperaturen zwischen −20°C und +70°C, vorzugsweise bei Temperaturen zwischen 0°C und +50°C.

Zur Durchführung des erfindungsgemäßen Verfahrens (d) setzt man pro Mol an 1-Aryl-pyrazol der Formel (Ib) im allgemeinen 0.8 bis 1.2 Mol, vorzugsweise äquimolare Mengen Oxidationsmittel der Formel (VIII) ein, wenn man die Oxidation des Schwefels auf der Sulfoxidstufe unterbrechen will. Zur Oxidation zum Sulfon setzt man pro Mol an 1-Aryl-pyrazol der Formel (Ib) im allgemeine 1.8 bis 3.0 Mol, vorzugsweise doppelt molare Mengen an Oxidationsmittel der Formel (VIII) ein Die Reaktionsführung, Aufarbeitung und Isolierung der Endprodukte der Formel (Ib) erfolgt nach üblichen Verfahren.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen Sie sind gegen normal sensible und resistente Arten sowie gegen alle oder einzelne Entwicklungsstadien wirksam Zu den oben erwähnten Schädlingen gehören :

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.

Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusiani, Caprocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchhidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räubemilben, Laufmilben, Fliegen (stechend und leckend), parasitierende Fliegenlarven, Läuse, Haarlinge, Federlinge, Flöhe.

Sie sind gegen normalsensible und resistente Arten und Stämme, sowie gegen alle parasitierenden und nicht parasitierenden Entwicklungsstadien der Ektoparasiten wirksam.

Die erfindungsgemäß verwendbaren Wirkstoffe der Formel (I) zeichnen sich durch eine hohe insektizide und akarizide Wirksamkeit aus. Sie lassen sich insbesondere gegen pflanzenschädigende Insekten, wie beispielsweise gegen die Raupen der Kohlschaube (Plutella maculipennis) oder gegen die Larven der Meerrettichblattkäfer (Phaedon cochleariae), sowie gegen pflanzenschädigende Milben, wie beispielsweise gegen die gemeine Spinnmilbe (Tetranychus urticae) einsetzen. Sie eignen sich daneben hervorragend zur Bekämpfung

von Bodeninsekten und lassen sich beispielsweise zur Bekämpfung von Phorbia antiqua-Maden einsetzen Auch eine nennenswerte wurzelsystemische Wirkung, beispielsweise gegen Phaedon cochleariae-Larven und Myzus persicae ist hervorzuheben.

Außerdem besitzen die erfindungsgemäß verwendbaren Wirkstoffe der Formel (I) eine hohe Wirkung gegen Hygiene und Vorratsschädlinge und lassen sich beispielsweise zur Bekämpfung der deutschen Schabe (Blatta germanica) oder zur Bekämpfung des gemeinen Kornkäfers (Sitophilus granarius) einsetzen Darüber hinaus lassen sich die erfindungsgemäßen Wirkstoffe mit besonders gutem erfolg zur Bekämpfung von parasitisch lebenden Warmblüterschädlingen, wie beispielsweise gegen Räudemilben (Psoroptes ovis), gegen Stechfliegen (Stomoxys calcitrans) oder gegen die Weideviehfliege (Musca autumnalis) einsetzen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche beinormaler Temperatur und unter Normaldruck gasförmig sind, z.B. Aerosol-Treibgas, wie Halogenkohlenwasserstoffe sowie Butan, Propan, Stickstoff und Kohlendioxid ; als feste Trägerstoffe kommen in Frage : z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage : z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate ; als Dispergiermittel kommen in Frage : z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe stoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäß verwendbaren Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäß verwendbaren Wirkstoffe geschieht auf diesem Gebiet in bekannter Weise wie durch orale Anwendung in Form von beispielweise Tabletten, Kapseln, Tränken, Granulaten, durch dermale bzw. äußerliche Anwendung in Form beispielsweise des Tauchens (Dippen), Sprühens (Sprayen), Aufgießens (pour-on and spot-on) und des Einpuderns sowie durch parenteiale Anwendung in Form beispielsweise der Injektion sowie ferner durch das "feed-through" -Verfahren Daneben ist auch eine Anwendung als Formkörper (Halsband, Ohrmarke) möglich.

Die biologische Wirksamkeit der erfindungsgemäßen Verbindungen soll anhand der folgenden Beispiele erläutert werden :

Herstellungsbeispiele :

Beispiel 1 :

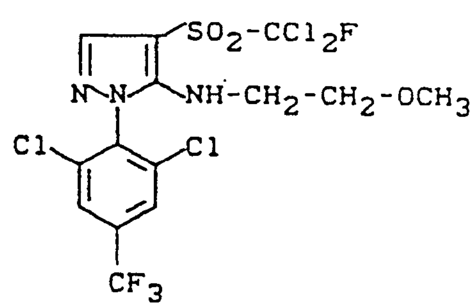

(Verfahren a)

32 g (0.06 Mol) 5-Brom-4-dichlorfluormethansulfonyl-1-(2,6-dichlor-4-trifluormethyl-phenyl)-pyrazol und 40 g (0.53 Mol) 2-Methoxy-ethylamin werden in 100 ml Dioxan gelöst und 24 Stunden bei 80°C gerührt. Nach Abkühlen wird die Reaktionsmischung in Wasser eingegossen und anschliessend mit Toluol extrahiert. Die Toluol-Phase wird mehrmals mit Wasser gewaschen, über Natriumsulfat getrocknet und im Vakuum eingeengt Der kristalline Rückstand wird aus Ligroin umkristallisiert.

Man erhält 17 g (55% der Theorie) 4-Dichlorfluormethansulfonyl-5-(2-methoxyethyl)-amino-1-(2,6-dichlor-4-trifluormethyl-phenyl)-pyrazol vom Schmelzpunkt 91°C-94°C

Herstellung der Ausgangsverbindung :

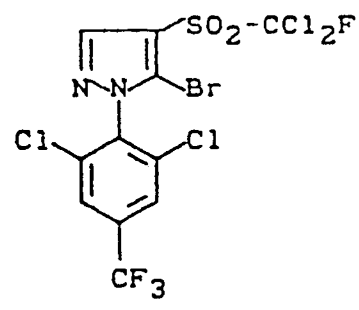

Zu einer Lösung von 72 g tertiärem Butylnitrit (0.7 Mol) in 400 ml Bromoform werden unter Rühren und Kühlen 98 g (0.21 Mol) 5-Amino-4-dichlorfluormethansulfonyl-1-(2,6-dichlor-4-trifluormethyl-phenyl)-pyrazol

portionsweise zugegeben. Die Temperatur wird zwischen 25°C und 30°C gehalten Nach 12-stündigem Rühren wird die Lösung am Rotationsverdampfer eingeengt und der Rückstand mit Petrolether kristallisiert.

Man erhält 95,4 g (85% der Theorie) 5-Brom-4-dichlorfluormethansulfonyl)-1-(2,6-dichlor-4-trifluormethyl-phenyl)-pyrazol vom Schmelzpunkt 97°C-98°C.

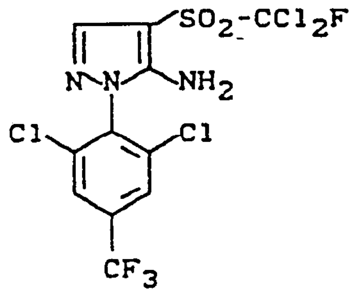

30 g (0.07 Mol) 5-Amino-4-dichlorfluormethansulfenyl-1-(2,6-dichlor-4-trifluormethyl-phenyl)-pyrazol in 200 ml Dichlormethan werden portionsweise mit 32 g (0.17 Mol) ca. 90-prozentiger m-Chlorperbenzoesäure versetzt, ca. 30 Stunden bei Raumtemperatur gerührt, filtriert, das Filtrat nacheinander mit gesättigter wässriger Natriumhydrogencarbonat-, gesättigter wässriger Natriumthiosulfat- und nochmals mit gesättigter wässriger Natriumhydrogensulfatlösung gewaschen, über Magnesiumsulfat getrocknet und im Vakuum vom Lösungsmittel befreit.

Man erhält 26,3 g (81,5% der Theorie) 5-Amino-4-dichlorfluormethylsulfonyl-1-(2,6-dichlor-4-trifluormethyl-phenyl)-pyrazol vom Schmelzpunkt 132°C-135°C.

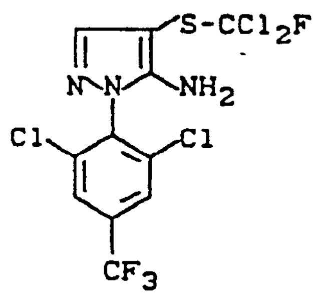

10 g (0.034 Mol) 5-Amino-1-(2,6-dichlor-4-trifluormethyl)-pyrazol werden in 50 ml Eisessig gelöst und bei Raumtemperatur tropfenweise mit 6,1 g (0.036 Mol) Dichlorfluormethansulfenylchlorid versetzt. Die Temperatur steigt bis auf ca. 40°C an Die Reaktionsmischung wird 2 Stunden gerührt und danach in eine Mischung aus 200 ml Wasser und 50 ml Dichlormethan eingetragen Die organische Phase wird abgetrennt, die wässrige mit zweimal 20 ml Dichlormethan extrahiert. Die vereinigten organischen Phasen werden nacheinander mit Natriumhydrogencarbonat- und Kochsalzlösung gewaschen, über Magnesiumsulfat getrocknet und im Vakuum eingeengt.

Man erhält 13,6 g (94% der Theorie) an 5-Amino-4-dichlorfluormethansulfenyl-1-(2,6-dichlor-4-trifluormethyl-phenyl)-pyrazol vom Schmelzpunkt 100°C-103°C.

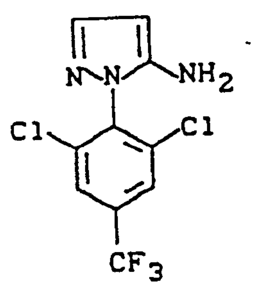

24,5 g (0.1 Mol) 2,6-Dichlor-4-trifluormethylphenylhydrazin und mit 20 mg Ethylendiamin-tetraessigsäure-Dinatriumsalz (= Titriplex III) in 150 ml Methanol werden bei Rückflußtemperatur tropfenweise mit 25 ml (27,6 g/0.3 Mol) 2-Chlor-acrylnitril versetzt Nach beendeter Zugabe erhitzt man weitere 8 Stunden auf Rückflußtemperatur, tropft dann 9 ml (0.16 Mol) 96%ige Schwefelsäure zu und erhitzt weitere 6 Stunden auf Rückflußtempeeatur. Die abgekühlte Reaktionsmischung wird mit 33,5 g (0.3 Mol) wasserfreiem Natriumcarbonat versetzt. Nach 4 Stunden entfernt man das Lösungsmittel im Vakuum, nimmt den Rückstand in 500 ml Wasser auf und rührt 10 Stunden bei Raumtemperatur. Man filtriert den ausgefallenen Niederschlag ab, wäscht mit Wasser nach und trocknet im Vakuum bei 50°C.

Man erhält 28,5 g (96% der Theorie) 5-Amino-1-(2,6-dichlor-4-trifluormethylphenyl)-pyrazol vom Schmelzpunkt 103°C-105°C.

Beispiel 2 :

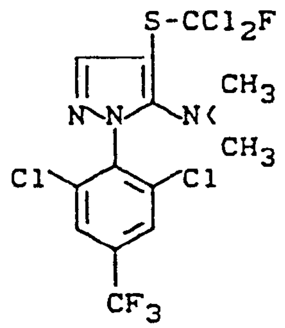

(Verfahren b)

30 g (0.07 Mol) 5-Amino-4-dichlorfluormethansulfenyl-1-(2,6-dichlor-4-trifluormethyl-phenyl)-pyrazol werden in 550 ml Dichlormethan gelöst und nacheinander mit 150 ml 40-prozentiger wässriger Natronlauge, drei Spatelspitzen Tributylbenzylammoniumchlorid und 21 ml (0.22 Mol) 97-prozentigem Dimethylsulfat versetzt und 16 Stunden bei Raumtemperatur gerührt. Die wässrige Phase wird abgetrennt, die organische mit Wasser gewaschen und im Vakuum vom Lösungsmittel befreit. Der Rückstand wird in 300 ml Ethanol aufgenommen, mit 30 ml 25-prozentigem wässrigen Ammoniak versetzt und 5 bis 10 Stunden gerührt. Danach wird das Lösungsmittel im Vakuum entfernt, der Rückstand in 300 ml Dichlormethan gelöst und die organische Phase nacheinander mit wässriger Ammoniumchlorid- und Kochsalzlösung gewaschen, über Magnesiumsulfat getrocknet und im Vakuum eingeengt.

Man erhält 31,5 g (98,6% der Theorie) an 4-Dichlorfluormethansulfenyl-5-dimethylamino-1-(2,6-dichlor-4-trifluormethyl-phenyl)-pyrazol vom Schmelzpunkt 85°C-89°C.

In entsprechender Weise und gemäß den allgemeinen Angaben zur Herstellung erhält man die folgenden 1-Aryl-pyrazole der allgemeinen Formel (I)

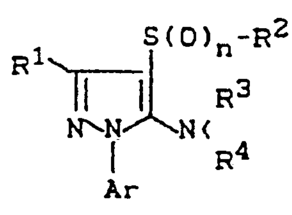

(I)

| Bsp. Nr. | $R^1$ | $-S(O)_n-R^2$ | $-N\langle^{R^3}_{R^4}$ | Ar | Schmelzpunkt/° C |
|---|---|---|---|---|---|
| 3 | H | $-SO-CCl_2F$ | $-N(CH_3)_2$ | 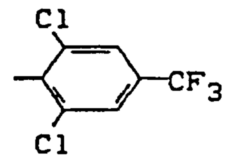 | $^1$H-NMR*): 2.83 |
| 4 | H | $-SO_2-CCl_2F$ | $-N(CH_3)_2$ | 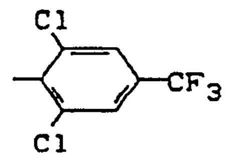 | $^1$H-NMR*): 2.86 |
| 5 | H | $-S-CClF_2$ | $-N(CH_3)_2$ | 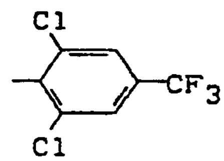 | Fp. 73 °C-74 °C |
| 6 | H | $-S-CF_3$ | $-N(CH_3)_2$ | 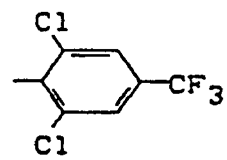 | Fp. 32 °C-36 °C |

*) Die $^1$H-NMR-Spektren wurden in $CDCl_3$ mit Tetramethylsilan (TMS) als innerem Standard aufgenommen. Angegeben ist die chemische Verschiebung als δ-Wert in ppm.

| Bsp. Nr. | $R^1$ | $-S(O)_n-R^2$ | $-N\langle^{R^3}_{R^4}$ | Ar | Schmelz-punkt/° C |
|---|---|---|---|---|---|
| 7 | H | $-SO_2-CCl_2F$ | $-NH-(CH_2)_2-OCH_3$ | 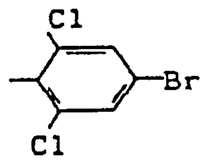 | |
| 8 | $-CH_3$ | $-SCF_3$ | $-N(CH_3)_2$ | 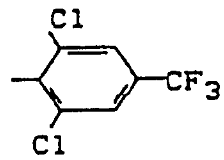 | Öl |
| 9 | $-CH_3$ | $-SCF_3$ | $-N(CH_3)_2$ | 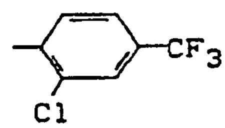 | Öl |
| 10 | $-CH_3$ | $-SCCl_2F$ | $-N(CH_3)_2$ | 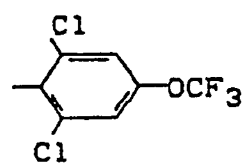 | Öl |
| 11 | $-CH_3$ | $-SCClF_2$ | $-N(CH_3)_2$ | 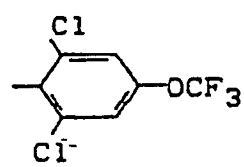 | Öl |
| 12 | $-CH_3$ | $-SCF_3$ | $-N(CH_3)_2$ | 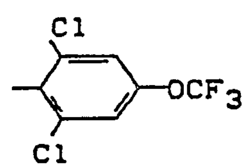 | Öl |
| 13 | $-CH_3$ | $-SCCl_2F$ | $-N(CH_3)_2$ | 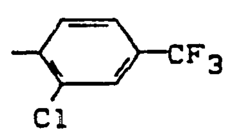 | Öl |
| 14 | $-CH_3$ | $-SCClF_2$ | $-N(CH_3)_2$ | 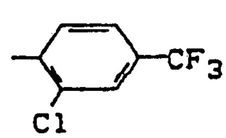 | Öl |

| Bsp. Nr. | $R^1$ | $-S(O)_n-R^2$ | $-N{<}^{R^3}_{R^4}$ | Ar | Schmelzpunkt/°C |
|---|---|---|---|---|---|
| 15 | $-CH_3$ | $-SCCl_2F$ | $-N(CH_3)_2$ | 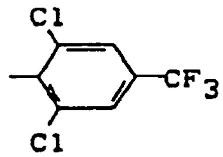 | Öl |
| 16 | $-CH_3$ | $-SCClF_2$ | $-N(CH_3)_2$ | 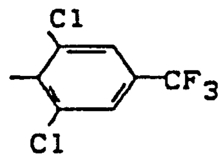 | Öl |
| 17 | H | $-SCF_3$ | $-NH-CH_2-C_6H_5$ | 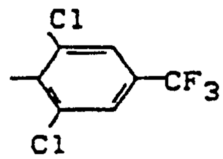 | 84 |
| 18 | H | $-SCCl_2F$ | $-NH-CH_2-C_6H_5$ | 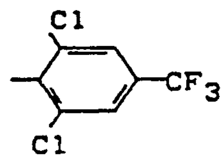 | 97 |
| 19 | $CH_3$ | $-S(O)CCl_2F$ | $-N(CH_3)_2$ | 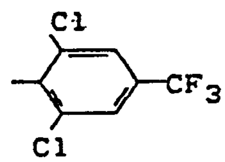 | 95-97 |
| 20 | $CH_3$ | $-SO_2-CCl_2F$ | $-N(CH_3)_2$ | 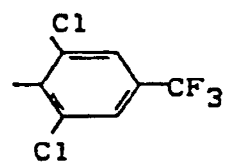 | 98-99 |
| 21 | $CH_3$ | $-S(O)CF_3$ | $-N(CH_3)_2$ | 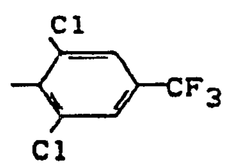 | 60-62 |

| Bsp. Nr. | $R^1$ | $-S(O)_n-R^2$ | $-N{<}^{R^3}_{R^4}$ | Ar | Schmelzpunkt/°C |
|---|---|---|---|---|---|
| 22 | H | $-SCCl_2F$ | $-N(C_2H_5)_2$ | 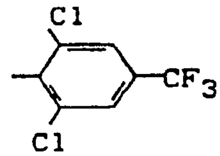 | $n_D^{20}$ :** 1.5350 |
| 23 | H | $-SCCl_2F$ | 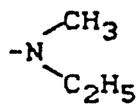 | 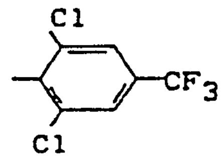 | 65-71 |
| 24 | H | $-SCF_3$ | $-N(C_2H_5)_2$ | 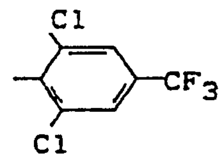 | $n_D^{20}$ : 1.5057 |
| 25 | H | $-SCF_3$ | 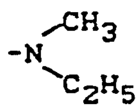 | 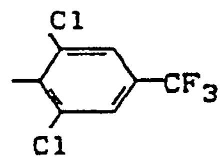 | Öl |
| 26 | H | $-SO_2CF_3$ | 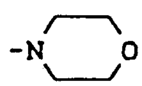 | 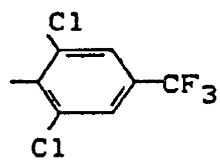 | 145-146 |
| 27 | H | $-SO_2-CCl_2F$ | 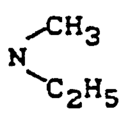 | 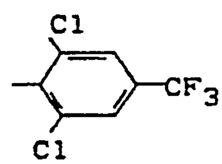 | 92-95 |

** $n_D^{20}$ : Brechnungsindex bei der Spektallinie des gelben Natriumlichts (D-Linie, 589 nm) und bei 20° C

Anwendungsbeispiele :

In den folgenden Anwendungsbeispielen wurden die nachstehend aufgeführten Verbindungen als Vergleichssubstanzen eingesetzt :

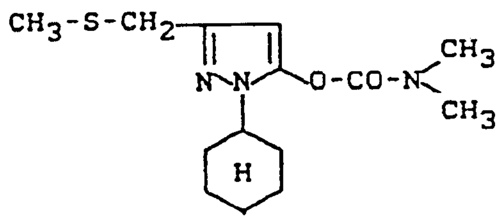

(A)

1-Cyclohexyl-5-[N,N-(dimethyl)-carbamoyloxy]-3-methylthiomethyl-pyrazol (bekannt aus DE-OS 2 839 270)

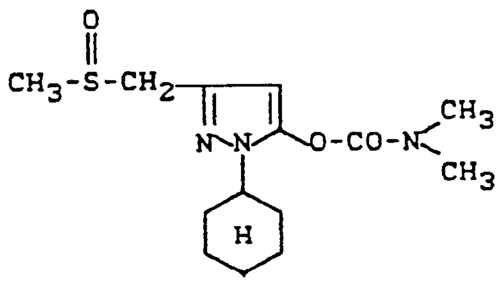

(B)

1-Cyclohexyl-5-(N,N-(dimethyl)-carbamoyloxy]-3-methylsulfinylmethyl-pyrazol (bekannt aus DE-OS 2 839 270)

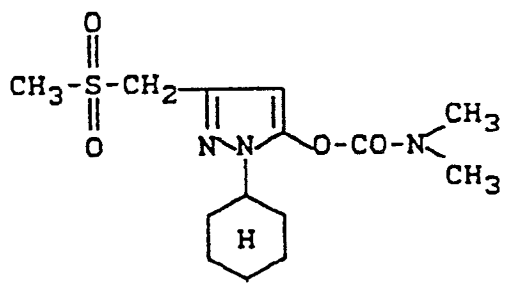

(C)

1-Cyclohexyl-5-(N,N-(dimethyl)-carbamoyloxy]-3-methylsulfonylmethyl-pyrazol (bekannt aus DE-OS 2 839 270).

<u>Beispiel A</u>

Phaedon-Larven-Test

| | |
|---|---|
| Lösungsmittel : | 3 Gewichtsteile |
| Emulgator : | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100%, daß alle Käferlarven abgetötet wurden ; 0% bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik : (2), (5), (1) und (7).

Beispiel B

Plutella-Test

| | |
|---|---|
| Lösungsmittel : | 3 Gewichtsteile |
| Emulgator : | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100%, daß alle Raupen abgetötet wurden ; 0% bedeutet, daß keine Raupen abgetötet wurden.

Bei diesem Test zeigen z.B. die folgenden Verbindungen der Herstellungsbeispiele überlegene Wirksamkeit gegenüber dem Stand der Technik : (1), (2), (3), (4), (5) und (7).

Beispiel C

Tetranychus-Test (resistent)

| | |
|---|---|
| Lösungsmittel : | 3 Gewichtsteile |
| Emulgator : | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100%, daß alle Spinnmilben abgetötet wurden ; 0% bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik : (1), (3), (4) und (7).

Beispiel D

Grenzkonzentrations-Test/Bodeninsekten

| | |
|---|---|
| Testinsekt : | Phorbia antigua-Maden im Boden |
| Lösungsmittel : | 3 Gewichtsteile Aceton |
| Emulgator : | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100%, wenn alle Testinsekten abgetötet worden sind, er ist 0%, wenn noch genau so viele Testinsekten leben wie bei der unbehandelten Kontrolle.

Bei diesem Test zeigen z.B. die folgende Verbindungen der Herstellungsbeispiele überlegene Wirkung gegenüber dem Stand der Technik : (2), (3), (4), (5), (12), (13), (14), (15), (16), (22), (23).

Beispiel E

Grenzkonzentrations-Test/Wurzelsystemische Wirkung

| | |
|---|---|
| Testinsekt : | Phaedon cochleariae -Larven |
| Lösungsmittel : | 3 Gewichtsteile Aceton |
| Emulgator : | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100%, wenn alle Testtiere abgetötet sind und 0%, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik : (2), (3), (4), (15), (23).

Beispiel F

Grenzkonzentrations-Test/Wurzelsystemische Wirkung

| | |
|---|---|
| Testinsekt : | Myzus persicae |
| Lösungsmittel : | 3 Gewichtsteile Aceton |
| Emulgator : | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die ängegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100%, wenn alle Testtiere abgetötet sind und 0%, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik : (2).

Beispiel G

$LD_{100}$-Test

| | |
|---|---|
| Testtiere : | Sitophilus granarius |
| Lösungsmittel : | Aceton |

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich

ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird 3 Tage nach Ansetzen der Versuche kontrolliert. Bestimmt wird die Abtötung in %. Dabei bedeutet 100%, daß alle Testtiere abgetötet wurden ; 0% bedeutet, daß keine Testtiere abgetötet wurden.

Bei diesem Test zeigen z.B. die folgenden Verbindungen der Herstellungsbeispiele überlegene Wirkung gegenüber dem Stand der Technik : (1), (2), (3) und (5).

Beispiel H
$LD_{100}$-Test

| | |
|---|---|
| Testtiere : | Blattella germanica |
| Lösungsmittel : | Aceton |

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und-bedeckt sie mit einem Glasdeckel.

Der Zustand der Testtiere wird 3 Tage nach Ansetzen der Versuche kontrolliert. Bestimmt wird die Abtötung in %. Dabei bedeutet 100%, daß alle Testtiere abgetötet wurden ; 0% bedeutet, daß keine Testtiere abgetötet wurden.

Bei diesem Test zeigen z.B. die folgenden Verbindungen der Herstellungsbeispiele überlegene Wirkung gegenüber dem Stand der Technik : (2) und (5).

Beispiel : I

Test mit Psoroptes ovis

Lösungsmittel : 35 Gewichtsteile Ethylenglykolmonomethylether 35 Gewichtsteile Nonylphenolpolyglykolether

Zur Herstellung Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnut das so erhaltene Konzentrat mit Wasser auf die gewünschte Konzentration,

Etwa 10-25 Psoroptes ovis werden in 1 ml der zu testenden Wirkstoffzubereitung gebracht, die in Tabellettennester einer Tiefziehpackung pipettiert wurden. Nach 24 Stunden wird der Abtötungsgrad bestimmt.

Bei diesem Test zeigt z. B. die folgende Verbindung der Herstellungsbeispiele überlegene Wirkung gegenüber dem Stand der Technik : (1).

Beispiel : K

Facefly-Test (Musca autumnalis)

Lösungsmittel : 35 Gewichtsteile Ethylenglykolmonomethylether 35 Gewichtsteile Nonylphenolpolyglykolether

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

10 adulte Faceflies (Musca autumnalis) werden in Petrischalen gebracht, die Filterpapierscheiben entsprechender Größe enthalten, die einen Tag vor Versuchsbeginn mit 1 ml der zu testenden Wirkstoffzubereitung durchtränkt wurden Nach 3 Stunden wird der Abtötungsgrad in Prozent bestimmt, wobei 100% bedeuten, daß alle und 0%, daß keine Fliegen abgetötet worden sind.

Bei diesem Test zeigt z.B. die folgende Verbindung der Herstellungsbeispiele überlegene Wirkung gegenüber dem Stand der Technik : (2).

Beispiel L

Test mit parasitierenden, adulten Stechfliegen (Stomoxys calcitrans)

Lösungsmittel : Cremophor

Zur Herstellung einer zweckmäßigen Wirkstoffzubereitung vermischt man die betreffende aktive Substanz mit dem angegebenen Lösungsmittel im Verhältnis 1 : 2 und verdünnt das so erhaltene Konzentrat mit Wasser auf die gewünschte Konzentration.

10 adulte Stechfliegen (Stomoxys calcitrans) werden in Petrischalen, die Sandwiches enthalten, die einen Tag vor Versuchsbeginn mit 1 ml der zu testenden Wirkstoffzubereitung durchtränkt wurden, gebracht, Nach 3 Stunden wird der Abtötungsgrad bestimmt, wobei 100% bedeuten, daO alle und 0%, daß keine Fliege abgetötet worden ist.

Bei diesem Test zeigt z.B. die folgende Verbindunge der Herstellungsbeispiele überlegene Wirkung gegenüber dem Stand der Technik : (2).

Beispiel M

Grenzkonzentrationstest/Bodeninsekten

| | |
|---|---|
| Testinsekt : | Diabrotica balteata-Larven im Boden |
| Lösungsmittel : | 3 Gewichtsteile Aceton |
| Emulgator : | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebne Menge Emulgator zu und verdünnt das Konzentrat mit Wasser auf die gewünschte Konzentration. Die Wirkstoffzubereitung wird innig mit dem Boden vermischt. Dabei spielt die Konzentration des Wirkstoffes in der Zubereitung praktisch keine Rolle, entsprechend ist allein die Wirkstoffgewichtsmenge pro Volumeneinheit Boden, welche in ppm (mg/l) angegeben wird. Man füllt den Boden in 0,5 l Töpfe und läßt diese bei 20°C stehen.

Sofort nach dem Ansatz werden je Topf 6 vorgekeimte Maiskörner gelegt. Nach 2 Tagen werden in den behandelten Boden die entsprechenden Testinsekten gesetzt. Nach weiteren 7 Tagen wird der Wirkungsgrad des Wirkstoffes durch Auszählen der toten und lebenden Testinsekten in % bestimmt. Der Wirkungsgrad ist 100%, wenn alle Testinsekten abgetötet worden sind, er ist 0%, wenn noch genau so viele Testinsekten leben wie in der unbehandelten Kontrolle.

Bei diesem Test zeigen z.B. die folgenden Verbindungen der Herstellungsbeispiele überlegene Wirkung gegenüber dem Stand der Tecnik : (3), (5), (12), (16).

## Ansprüche

1. 1-Aryl-pyrazole der allgemeinen Formel (I)

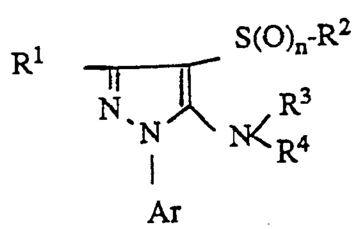

(I)

in welcher

$R^1$ für Wasserstoff, oder für jeweils geradkettiges oder verzweigtes Alkyl oder Halogenalkyl mit 1 bis 4 Kohlenstoffatomen und gegebenenfalls 1 bis 9 gleichen oder verschiedenen Halogenatomen steht,

$R^2$ für jeweils geradkettiges oder verzweigtes Alkyl, Alkenyl oder Alkinyl mit jeweils bis zu 8 Kohlenstoffatomen, für Cycloalkyl mit 3 bis 7 Kohlenstoffatomen, für jeweils geradkettiges oder verzweigtes Halogenalkyl oder Halogenalkenyl mit jeweils bis zu 8 Kohlenstoffatomen und bis zu 17 gleichen oder verschiedenen Halogenatomen, für jeweils geradkettiges oder verzweigtes Alkoxyalkyl, Alkylthioalkyl, Alkylsulfinylalkyl oder Alkylsulfonylalkyl mit jeweils 1 bis 6 Kohlenstoffatomen in den einzelnen Alkylteilen oder für jeweils im Phenylteil

gegebenenfalls einfach oder mehrfach, gleich oder verschieden substituiertes Phenylalkyl oder Phenyl mit gegebenenfalls 1 bis 4 Kohlenstoffatomen im geradkettigen oder verzweigten Alkylteil steht, wobei als Substituenten im Phenylteil jeweils infrage kommen : Halogen, Cyano, Nitro, jeweils geradkettiges oder verzweigtes Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen in den einzelnen Alkylteilen und gegebenenfalls 1 bis 9 gleichen oder verschiedenen Halogenatomen,

$R^3$ für jeweils geradkettiges oder verzweigtes Alkyl, Alkenyl, Alkinyl, Halogenalkyl oder Halogenalkenyl mit jeweils bis zu 8 Kohlenstoffatomen und gegebenenfalls mit 1 bis 17 Halogenatomen steht, außerdem für jeweils geradkettiges oder verzweigtes Alkoxyalkyl oder Alkylthioalkyl mit jeweils 1 bis 4 Kohlenstoffatomen in den einzelnen Alkylteilen, für Cycloalkyl mit 3 bis 7 Kohlenstoffatomen oder für im Phenylteil gegebenenfalls einfach- oder mehrfach, gleich oder verschieden substituiertes, im Alkylteil geradkettiges oder verzweigtes Phenylalkyl mit 1 bis 4 Kohlenstoffatomen im Alkylteil steht, wobei als Phenylsubstituenten die bei $R^2$ genannten infrage kommen,

$R^4$ für jeweils geradkettiges oder verzweigtes Alkyl, Alkenyl, Alkinyl, Halogenalkyl oder Halogenalkenyl mit jeweils bis zu 8 Kohlenstoffatomen und gegebenenfalls mit 1 bis 17 Halogenatomen steht, außerdem für jeweils geradkettiges oder verzweigtes Alkoxyalkyl oder Alkylthioalkyl mit jeweils 1 bis 4 Kohlenstoffatomen in den einzelnen Alkylteilen, für Cycloalkyl mit 3 bis 7 Kohlenstoffatomen oder für im Phenylteil gegebenenfalls einfach- oder mehrfach, gleich oder verschieden substituiertes, im Alkylteilgerad-kettiges oder verzweigtes Phenylalkyl mit 1 bis 4 Kohlenstoffatomen im Alkylteil steht, wobei als Phenylsubstituenten die bei $R^2$ genannten infrage kommen, und darüberhinaus auch für Wasserstoff steht, für den Fall, daß dann $R^3$ nicht gleichzeitig für unsubstituiertes Alkyl steht, oder

$R^3$ und $R^4$ gemeinsam mit dem Stickstoffatom, an welches sie gebunden sind, für einen 3- bis 7- gliedrigen gesättigten Heterocyclus stehen, der bis zu 2 weitere Heteroatome, insbesondere Stickstoff, Sauerstoff und/oder Schwefel enthalten kann,

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten jeweils infrage kommen : Cyano, Nitro, Halogen, jeweils geradkettiges oder verzweigtes Alkyl, Alkoxy oder Alkoxycarbonyl mit jeweils 1 bis 4 Kohlenstoffatomen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_p-R^6$ und

n für eine Zahl 0, 1 oder 2 steht, wobei

$R^6$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht und

p für eine Zahl 0, 1 oder 2 steht.

2. 1-Aryl-pyrazole gemäß Anspruch 1, dadurch gekennzeichnet, daß in der allgemeinen Formel (I)

$R^1$ für Wasserstoff, Methyl, Ethyl, n- oder i-Propyl und Trifluormethyl steht,

$R^2$ für Methyl, Ethyl, n- oder i-Propyl, n-, i-, s- oder t-Butyl, n- oder i-Pentyl, n- oder i-Hexyl, Allyl, n- oder i-Butenyl, n- oder i-Pentenyl, Propargyl, n-oder i-Butinyl, Cyclopropyl, Cyclobutyl, Cyclopentyl, Cyclohexyl, Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Chlorpropyl, Brompropyl, Dichlormethyl, Chlorfluormethyl, Trichlormethyl, Dichlormethyl, Trifluorethyl, Trifluorchlorethyl, Tetrafluorethyl, Difluorchlorethyl, Fluordibrommethyl, Difluorbrommethyl, Fluorchlorbrommethyl, Chlorallyl, Fluorallyl, Chlorbutenyl, Fluorbutenyl, Dichlorallyl, Fluorchlorallyl, Dichlorbutenyl, Difluorallyl, Bromallyl, für Methoxymethyl, Methoxyethyl, Methoxypropyl, Ethoxymethyl, Ethoxyethyl, Methylthiomethyl, Methylsulfinylmethyl, Methyl-sulfonylethyl, Methylthioethyl, Methylthiopropyl, Methylsulfinylethyl, Methylsulfonylmethyl oder für jeweils gegebenenfalls einfach bis dreifach, gleich oder verschieden substituiertes Phenyl, Benzyl oder Phenylethyl steht, wobei als Phenylsubstituenten jeweils in Frage kommen : Fluor, Chlor, Brom, Iod, Cyano, Nitro, Methyl, Ethyl, Methoxy, Methylthio, Trifluormethyl, Methylsulfinyl, Methylsulfonyl, Trifluormethoxy, Trifluormethylthio, Trifluormethylsulfinyl oder Trifluormethylsulfonyl,

$R^3$ für Methyl, Ethyl, n- oder i-Propyl, n-, i-, s- oder t-Butyl, n- oder i-Pentyl, n- oder i-Hexyl, für Allyl, n- oder i-Butenyl, n- oder i-Pentenyl, Propargyl, D- oder i-Butinyl, Cyclopropyl, Cyclobutyl, Cyclopentyl, Cyclohexyl, Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Chlorpropyl, Brompropyl, Dichlormethyl, Chlorfluormethyl, Trichlormethyl, Trifluorethyl, Trifluorchlorethyl, Tetrafluorethyl, Difluorchlorethyl, Fluordibrommethyl, Difluorbrommethyl, Fluorchlorbrommethyl, Chlorallyl, Fluorallyl, Chlorbutenyl, Fluorbutenyl, Dichlorallyl, Fluorchlorallyl, Dichlorbutenyl, Difluorallyl, Bromallyl, für Methoxymethyl, Methoxyethyl, Methoxypropyl, Ethoxymethyl, Ethoxyethyl, Methylthiomethyl, Methylthioethyl, Methylthiopropyl, Ethylthiomethyl, Ethylthioethyl oder für jeweils im Phenylteil gegebenenfalls

ein- bis dreifach, gleich oder verschieden substituiertes Benzyl oder Phenylethyl steht, wobei als Phenylsubstituenten die bei $R^2$ genannten infrage kommen,

$R^4$ für Methyl, Ethyl, n- oder i-Propyl, n-, i-, s- oder t-Butyl, n- oder i-Pentyl, n- oder i-Hexyl, für Allyl, n- oder i-Butenyl, n- oder i-Pentenyl, Propargyl, n- oder i-Butinyl, Cyclopropyl, Cyclobutyl, Cyclopentyl, Cyclohexyl, Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Chlorpropyl, Brompropyl, Dichlormethyl, Chlorfluormethyl, Trichlormethyl, Trifluorethyl, Trifluorchlorethyl, Tetrafluorethyl, Difluorchlorethyl, Fluordibrommethyl, Difluorbrommethyl, Fluorchlorbrommethyl, Chlorallyl, Fluorallyl, Chlorbutenyl, Fluorbutenyl, Dichlorallyl, Fluorchlorallyl, Dichlorbutenyl, Difluorallyl, Bromallyl, für Methoxymethyl, Methoxyethyl, Methoxypropyl, Ethoxymethyl, Ethoxyethyl, Methylthiomethyl, Methylthioethyl, Methylthiopropyl, Ethylthiomethyl, Ethylthioethyl oder für jeweils im Phenylteil gegebenenfalls ein- bis dreifach, gleich oder verschieden substituiertes Benzyl oder Phenylethyl steht, wobei als Phenylsubstituenten die bei $R^2$ genannten infrage kommen, und darüberhinaus auch für Wasserstoff steht, für den Fall, daß dann $R^3$ nicht gleichzeitig für unsubstituiertes Alkyl steht, oder

$R^3$ und $R^4$ gemeinsam mit dem Stickstoffatom, an welches sie gebunden sind, für Pyrrolidinyl, Piperidinyl, Perhydroazepinyl oder Morpholinyl stehen,

Ar für gegebenenfalls ein- bis fünffach, gleich oder verschieden substituiertes Phenyl oder für gegebenenfalls ein- bis vierfach, gleich oder verschieden substituiertes 2-Pyridyl steht, wobei als Substituenten jeweils infrage kommen : Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_p-R^6$ und

n für eine Zahl 0, 1 oder 2 steht, wobei

$R^6$ für Amino, Methylamino, Ethylamino, Dimethylamino, Diethylamino, Fluordichlormethyl, Difluormethyl, Tetrafluorethyl, Trifluorchlorethyl, Trichlormethyl, Trichlorethyl, Trifluormethyl, Methyl oder Ethyl steht und

p für eine Zahl 0, 1 oder 2 steht.

3. Verfahren zur Herstellung von 1-Aryl-pyrazolen der allgemeinen Formel (I)

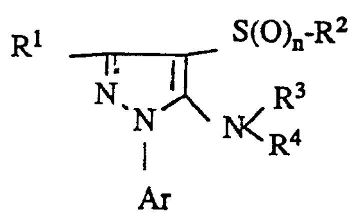

(I)

in welcher

$R^1$ für Wasserstoff, oder für jeweils geradkettiges oder verzweigtes Alkyl oder Halogenalkyl mit 1 bis 4 Kohlenstoffatomen und gegebenenfalls 1 bis 9 gleichen oder verschiedenen Halogenatomen steht,

$R^2$ für jeweils geradkettiges oder verzweigtes Alkyl, Alkenyl oder Alkinyl mit jeweils bis zu 8 Kohlenstoffatomen, für Cycloalkyl mit 3 bis 7 Kohlenstoffatomen, für jeweils geradkettiges oder verzweigtes Halogenalkyl oder Halogenalkenyl mit jeweils bis zu 8 Kohlenstoffatomen und bis zu 17 gleichen oder verschiedenen Halogenatomen, für jeweils geradkettiges oder verzweigtes Alkoxyalkyl, Alkylthioalkyl, Alkylsulfinylalkyl oder Alkylsulfonylalkyl mit jeweils 1 bis 6 Kohlenstoffatomen in den einzelnen Alkylteilen oder für jeweils im Phenylteil gegebenenfalls einfach oder mehrfach, gleich oder verschieden substituiertes Phenylalkyl oder Phenyl mit gegebenenfalls 1 bis 4 Kohlenstoffatomen im geradkettigen oder verzweigten Alkylteil steht, wobei als Substituenten im Phenylteil jeweils infrage kommen : Halogen, Cyano, Nitro, jeweils geradkettiges oder verzweigtes Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen in den einzelnen Alkylteilen und gegebenenfalls 1 bis 9 gleichen oder verschiedenen Halogenatomen,

$R^3$ für jeweils geradkettiges oder verzweigtes Alkyl, Alkenyl, Alkinyl, Halogenalkyl oder Halogenalkenyl mit jeweils bis zu 8 Kohlenstoffatomen und gegebenenfalls mit 1 bis 17 Halogenatomen steht, außerdem für jeweils geradkettiges oder verzweigtes Alkoxyalkyl oder Alkylthioalkyl mit jeweils 1 bis 4 Kohlenstoffatomen in den einzelnen Alkylteilen, für Cycloalkyl mit 3 bis 7 Kohlenstoffatomen oder für im Phenylteil gegebenenfalls einfach- oder mehrfach, gleich oder verschieden substituiertes, im Alkylteil geradkettiges oder verzweigtes Phenylalkyl

mit 1 bis 4 Kohlenstoffatomen im Alkylteil steht, wobei als Phenylsubstituenten die bei $R^2$ genannten infrage kommen,

$R^4$ für jeweils geradkettiges oder verzweigtes Alkyl, Alkenyl, Alkinyl, Halogenalkyl oder Halogenalkenyl mit jeweils bis zu 8 Kohlenstoffatomen und gegebenenfalls mit 1 bis 17 Halogenatomen steht, außerdem für jeweils geradkettiges oder verzweigtes Alkoxyalkyl oder Alkylthioalkyl mit jeweils 1 bis 4 Kohlenstoffatomen in den einzelnen Alkylteilen, für Cycloalkyl mit 3 bis 7 Kohlenstoffatomen oder für im Phenylteil gegebenenfalls einfach- oder mehrfach, gleich oder verschieden substituiertes, im Alkylteilgerad-kettiges oder verzweigtes Phenylalkyl mit 1 bis 4 Kohlenstoffatomen im Alkylteil steht, wobei als Phenylsubstituenten die bei $R^2$ genannten infrage kommen, und darüberhinaus auch für Wasserstoff steht, für den Fall, daß dann $R^3$ nicht gleichzeitig für unsubstituiertes Alkyl steht, oder

$R^3$ und $R^4$ gemeinsam mit dem Stickstoffatom, an welches sie gebunden sind, für einen 3- bis 7- gliedrigen gesättigten Heterocyclus stehen, der bis zu 2 weitere Heteroatome, insbesondere Stickstoff, Sauerstoff und-/oder Schwefel enthalten kann,

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten jeweils infrage kommen : Cyano, Nitro, Halogen, jeweils geradkettiges oder verzweigtes Alkyl, Alkoxy oder Alkoxycarbonyl mit jeweils 1 bis 4 Kohlenstoffatomen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_p-R^6$ und

n für eine Zahl 0, 1 oder 2 steht, wobei

$R^6$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht und

p für eine Zahl 0, 1 oder 2 steht, dadurch gekennzeichnet, daß man

(a) 5-Halogen-1-aryl-pyrazole der Formel (II),

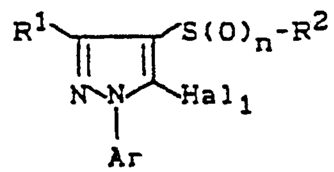

(II)

in welcher

$R^1$, $R^2$, n und Ar die oben angegebene Bedeutung haben und

$Hal^1$ für Halogen steht, mit Aminen der Formel (III),

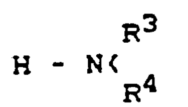

(III)

in welcher

$R^3$ und $R^4$ die oben angegebene Bedeutung haben, gegebenenfalls in Gegenwart eines Verdünnungsmittels und gegebenenfalls in Gegenwart eines Säurebindemittels umsetzt, oder daß man

(b) 5-Amino-1-aryl-pyrazole der formel

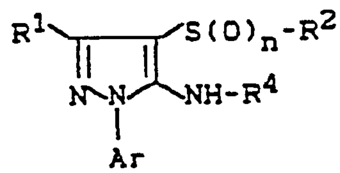

(IV)

in welcher

$R^1$, $R^2$, $R^4$, n und Ar die oben angegebene Bedeutung haben, mit Alkylierungsmitteln der formel (V),

$$R^3\text{-}E^1 \quad (V)$$

in welcher
$R^3$ die oben angegebene Bedeutung hat und
$E^1$ für eine elektronenanziehende Abgangsgruppe steht, gegebenenfalls in Gegenwart eines Verdünnungsmittels, gegebenenfalls in Gegenwart eines Säurebindemittels und gegebenenfalls in Gegenwart eines Katalysators umsetzt ;
oder daß man zum Erhalt von 1-Aryl-pyrazolen der Formel (Ia),

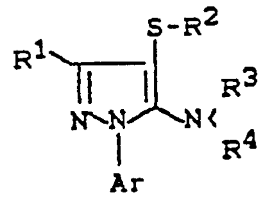

(Ia)

in welcher
$R^1$, $R^2$, $R^3$, $R^4$ und Ar die oben angegebene Bedeutung haben,
(c) 4-unsubstituierte 1-Aryl-pyrazole der Formel (VI),

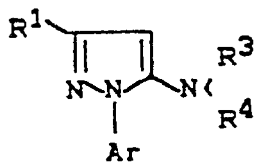

(IV)

in welcher
$R^1$, $R^3$, $R^4$ und Ar die oben angegebene Bedeutung haben, mit Sulfenylhalogeniden der Formel (VII),

$R^2$-S-$Hal^2$ (VII)

in welcher
$R^2$ die oben angegebene Bedeutung hat und
$Hal^2$ für Halogen steht, gegebenenfalls in Gegenwart eines Verdünnungsmittels und gegebenenfalls in Gegenwart eines Säurebindemittels umsetzt ;
oder daß man zum Erhalt von 1-Aryl-pyrazolen der Formel (Ib),

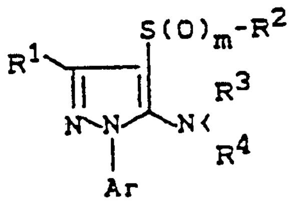

(Ib)

in welcher
$R^1$, $R^2$, $R^3$, $R^4$ und Ar die oben angegebene Bedeutung haben und
m für eine Zahl 1 oder 2 steht,
(d) die nach Verfahren (a), (b) oder (c) erhältlichen 1-Aryl-pyrazole der Formel (Ia)

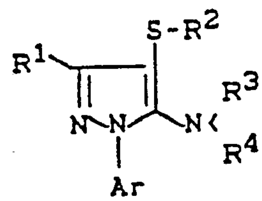

(Ia)

in welcher
$R^1$, $R^2$, $R^3$, $R^4$ und Ar die oben angegebene Bedeutung haben mit Oxidationsmitteln der Formel (VIII),

$R^5$-O-O-H (VIII)

in welcher
$R^5$ für Wasserstoff oder für jeweils gegebenenfalls substituiertes Alkanoyl oder Aroyl steht, gegebenenfalls in Gegenwart eines Verdünnungsmittels, gegebenenfalls in Gegenwart eines Katalysators sowie gegebenenfalls in Gegenwart eines Säurebindemittels umsetzt.

4. Schädlingsbekämpfungsmittel, gekennzeichnet, durch einen Gehalt an mindestens einem 1-Aryl-pyrazol nach Anspruch 1.

5. Insektizide und akarizide Mittel, gekennzeichnet durch einen Gehalt an mindestens einem 1-Aryl-pyrazol nach Anspruch 1.

6. Verfahren zur Bekämpfung von Insekten und/oder Spinnentieren, dadurch gekennzeichnet, daß man 1-Aryl-pyrazole nach Anspruch 1 auf Insekten und/oder Spinnentiere und/oder deren Lebensraum einwirken läßt.

7. Verwendung von 1-Aryl-pyrazolen nach Anspruch 1 zur Bekämpfung von tierischen Schädlingen, insbesondere von Insekten und/oder Spinnentieren.

8. Verfahren zur Herstellung von Schädlingsbekämpfungsmitteln, dadurch gekennzeichnet, daß man 1-Aryl-pyrazole nach Anspruch 1 mit Streckmitteln und/oder oberflächenaktiven Mitteln vermischt.

**Claims**

1. 1-Aryl-pyrazoles of the general formula (I)

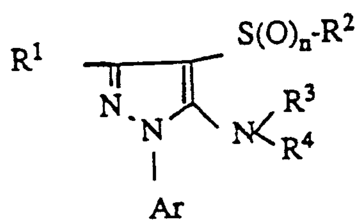

(I)

in which
$R^1$ represents hydrogen, or in each case straight-chain or branched alkyl or halogenoalkyl having 1 to 4 carbon atoms and, if appropriate, 1 to 9 identical or different halogen atoms,

$R^2$ represents in each case straight-chain or branched alkyl, alkenyl or alkinyl having up to 8 carbon atoms in each case, cycloalkyl having 3 to 7 carbon atoms, in each case straight-chain or branched halogenoalkyl or halogenoalkenyl having up to 8 carbon atoms in each case and up to 17 identical or different halogen atoms, in each case straight-chain or branched alkoxyalkyl, alkylthioalkyl, alkylsulphinylalkyl or alkylsulphonylalkyl having, in each case, 1 to 6 carbon atoms in the individual alkyl parts, or in each case optionally mono- or polysubstituted phenylalkyl or phenyl having, if appropriate, 1 to 4 carbon atoms in the straight-chain or branched alkyl part, the substituents in each case being in the phenyl part and being identical or different and suitable substituents in the phenyl part in each case being : halogen, cyano, nitro, in each case straight-chain or branched alkyl, alkoxy, alkylthio, alkylsulphinyl, alkylsulphonyl, halogenoalkyl, halogenoalkoxy, halogenoalkylthio, halogenoalkylsulphinyl or halogenoalkylsulphonyl in each case having 1 to 4 carbon atoms in the individual alkyl parts and, if appropriate, 1 to 9 identical or different halogen atoms,

$R^3$ represents in each case straight-chain or branched alkyl, alkenyl, alkinyl, halogenoalkyl or halogenoalkenyl, in each case having up to 8 carbon atoms and, if appropriate, having 1 to 17 halogen atoms, additionally represents in each case straight-chain or branched alkoxyalkyl or alkylthioalkyl in each case having 1 to 4 carbon atoms in the individual alkyl parts, cycloalkyl having 3 to 7 carbon atoms or optionally mono- or polysubstituted phenylalkyl, having 1 to 4 carbon atoms in the alkyl part and being straight-chain or branched in the alkyl part, the substitutents being in the phenyl part and being identical or different and suitable phenyl substituents being those mentioned in the case of $R^2$,

$R^4$ represents in each case straight-chain or branched alkyl, alkenyl, alkinyl, halogenoalkyl or halogenoalkenyl in each case having up to 8 carbon atoms and, if appropriate, 1 to 17 halogen atoms, additionally represents in each case straight-chain or branched alkoxyalkyl or alkylthioalkyl in each case having 1 to 4 carbon atoms in the individual alkyl parts, cycloalkyl having 3 to 7 carbon atoms, or optionally mono- or polysubstituted phenylalkyl, having 1 to 4 carbon atoms in the alkyl part and being straight-chain or branched in the alkyl part, the substituents being in the phenyl part and being identical or different and suitable phenyl substituents being those mentioned in the case of $R^2$, and in addition also represents hydrogen in the case where $R^3$ does not simultaneously represent unsubstituted alkyl, or

$R^3$ and $R^4$, together with the nitrogen atom to which they are bound, represent a 3- to 7-membered saturated heterocycle, which can contain up to 2 further heteroatoms, particularly nitrogen, oxygen and/or sulphur,

Ar represents in each case optionally mono- or polysubstituted phenyl, 2-pyridyl, 3-pyridyl or 4-pyridyl, the substituents being identical or different and suitable substituents in each case being : cyano, nitro, halogen, in each case straight-chain or branched alkyl, alkoxy or alkoxycarbonyl, in each case having 1 to 4 carbon atoms, in each case straight-chain or branched halogenoalkyl or halogenoalkoxy in each case having 1 to 4 carbon atoms and 1 to 9 identical or different halogen atoms, or a $-S(O)_p-R^6$ radical, and

n represents a number 0, 1 or 2, where

$R^6$ represents amino, and also in each case straight-chain or branched alkyl, alkylamino, dialkylamino or halogenoalkyl, in each case having up to 4 carbon atoms in the individual alkyl parts and, in the case of halogenoalkyl, having up to 9 identical or different halogen atoms, and

p represents a number 0, 1 or 2.

2. 1-Aryl-pyrazoles according to Claim 1, characterised in that, in the general formula (I)

$R^1$ represents hydrogen, methyl, ethyl, n- or i-propyl and trifluoromethyl,

$R^2$ represents methyl, ethyl, n- or i-propyl, n-, i-, s- or t-butyl, n- or i-pentyl, n- or i-hexyl, allyl, n- or i-butenyl, n- or i-pentenyl, propargyl, n- or i-butinyl, cyclopropyl, cyclobutyl, cyclopentyl, cyclohexyl, chloromethyl, difluoromethyl, difluorochloromethyl, fluorodichloromethyl, trifluoromethyl, pentafluoroethyl, pentachloroethyl, fluorotetrachloroethyl, difluorotrichloroethyl, trifluorodichloroethyl, tetrafluorochloroethyl, heptafluoropropyl, chloroethyl, bromoethyl, chloropropyl, bromopropyl, dichloromethyl, chlorofluoromethyl, trichloromethyl,dichloromethyl,trifluoromethyl, trifluorochloroethyl, tetrafluoroethyl, difluorochloroethyl, fluorodibromomethyl, difluorobromomethyl, fluorochlorobromomethyl, chloroallyl, fluoroallyl, chlorobutenyl, fluorobutenyl, dichloroallyl, fluorochloroallyl, dichlorobutenyl, difluoroallyl, bromoallyl, methoxymethyl, methoxyethyl, methoxypropyl, ethoxymethyl, ethoxyethyl, methylthiomethyl, methyl-sulphinylmethyl, methyl-sulphonylethyl, methylthioethyl, methylthiopropyl, methyl-sulphinylethyl, methylsulphonylmethyl or in each case optionally mono- to trisubstituted phenyl, benzyl or phenylethyl, the substituents being identical or different and suitable phenyl substituents in each case being : fluorine, chlorine, bromine, iodine, cyano, nitro, methyl, ethyl, methoxy, methylthio, trifluoromethyl, methylsulphinyl, methylsulphonyl, trifluoromethoxy, trifluoromethylthio, trifluoromethylsulphinyl or trifluoromethylsulphonyl,

$R^3$ represents methyl, ethyl, n- or i-propyl, n-, i-, s- or t-butyl, n- or i-pentyl, n- or i-hexyl, allyl, n- or i-butenyl, n- or i-pententyl, propargyl, n- or i-butinyl, cyclopropyl, cyclobutyl, cyclopentyl, cyclohexyl, chloromethyl, difluoromethyl, difluorochloromethyl, fluorodichloromethyl, trifluoromethyl, pentafluoroethyl, pentachloroethyl, fluorotetrachloroethyl, difluorotrichloroethyl, trifluorodichloroethyl, tetrafluorochloroethyl, heptafluoropropyl, chloroethyl, bromoethyl, chloropropyl, bromopropyl, dichloromethyl, chlorofluoromethyl, trichloromethyl, trifluoroethyl, trifluorochloroethyl, tetrafluoroethyl, difluorochloroethyl, fluorodibromomethyl, difluorobromomethyl, fluorochlorobromomethyl, chloroallyl, fluoroallyl, chlorobutenyl, fluorobutenyl, dichloroallyl, fluorochloroallyl, dichlorobutenyl, difluoroallyl, bromoallyl, methoxymethyl, methoxyethyl, methoxypropyl, methoxymethyl, ethoxyethyl, methylthiomethyl, methylthioethyl, methylthiopropyl, ethylthiomethyl, ethylthioethyl or in each case optionally mono- to trisubstituted benzyl or phenylethyl, the substituents being in the phenyl part and being identical or different and suitable phenyl substituents being those mentioned in the case of $R^2$,

$R^4$ represents methyl, ethyl, n- or i-propyl, n- i-, s- or t-butyl, n- or i-pentyl, n- or i-hexyl, allyl, n- or i-butenyl, n- or i-pentenyl, propargyl, n- or i-butinyl, cyclopropyl, cyclobutyl, cyclopentyl, cyclohexyl, chloromethyl, difluoromethyl, difluorochloromethyl, fluorodichloromethyl, trifluoromethyl, pentafluoroethyl, pentachloroethyl, fluorotetrachloroethyl, difluorotrichloroethyl, trifluorodichloroethyl, tetrafluorochloroethyl, heptafluoropropyl,

chloroethyl, bromoethyl, chloropropyl, bromopropyl, dichloromethyl, chlorofluoromethyl, trichloromethyl, trifluoroethyl, trifluorochloroethyl, tetrafluoroethyl, difluorochloroethyl, fluorodibromomethyl, difluorobromomethyl, fluorochlorobromomethyl, chloroallyl, fluoroallyl, chlorobutenyl, fluorobutenyl, dichloroallyl, fluorochloroallyl, dichlorobutenyl, difluoroallyl, bromoallyl, methoxymethyl, methoxyethyl, methoxypropyl, ethoxymethyl, ethoxyethyl, methylthiomethyl, methylthioethyl, methylthiopropyl, ethylthiomethyl, ethylthioethyl or in each case optionally mono- to trisubstituted benzyl or phenylethyl, the substituents being in the phenyl part and being identical or different and suitable phenyl substituents being those mentioned in the case of $R^2$, and, in addition, also represents hydrogen in the case where $R^3$ does not simultaneously represent unsubstituted alkyl, or $R^3$ and $R^4$, together with the nitrogen atom to which they are bound, represent pyrrolidinyl, piperidinyl, perhydroazepinyl or morpholinyl,

Ar represents optionally mono- to pentasubstituted phenyl, the substituents being identical or different, or optionally mono- to tetrasubstituted 2-pyridyl, the substituents being identical or different and suitable substituents in each case being : cyano, nitro, fluorine, chlorine, bromine, iodine, methyl, ethyl, n- and i-propyl, n-, i-, s- and t-butyl, methoxy, ethoxy, methoxycarbonyl, ethoxycarbonyl, trifluoromethyl, trichloromethyl, dichlorofluoromethyl, difluorochloromethyl, chloromethyl, dichloromethyl, difluoromethyl, pentafluoroethyl, tetrafluoroethyl, trifluorochloroethyl, trifluoroethyl, difluorodichloroethyl, trifluorodichloroethyl, pentachloroethyl, trifluoromethoxy, trichloromethoxy, dichlorofluoromethoxy, difluorochloromethoxy, chloromethoxy, dichloromethoxy, difluoromethoxy, pentafluoroethoxy, tetrafluoroethoxy, trifluorochloroethoxy, trifluoroethoxy,difluorodichloroethoxy, trifluorodichloroethoxy, pentachloroethoxy or an $-S(O)_p-R^6$ radical, and

n represents a number 0, 1 or 2, where

$R^6$ represents amino, methylamino, ethylamino, dimethylamino, diethylamino, fluorodichloromethyl, difluoromethyl, tetrafluoroethyl, trifluorochloroethyl, trichloromethyl, trichloroethyl, trifluoromethyl, methyl or ethyl, and

p represents a number 0, 1 or 2.

3. Process for the preparation of 1-aryl-pyrazoles of the general formula (I)

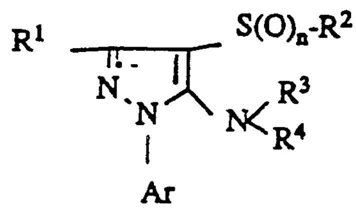

(I)

in which

$R^1$ represents hydrogen, or in each case straight-chain or branched alkyl or halogenoalkyl having 1 to 4 carbon atoms and, if appropriate, 1 to 9 identical or different halogen atoms,

$R^2$ represents in each case straight-chain or branched alkyl, alkenyl or alkinyl having up to 8 carbon atoms in each case, cycloalkyl having 3 to 7 carbon atoms, in each case straight-chain or branched halogenoalkyl or halogenoalkenyl having up to 8 carbon atoms in each case and up to 17 identical or different halogen atoms, in each case straight-chain or branched alkoxyalkyl, alkylthioalkyl, alkylsulphinylalkyl or alkylsulphonylalkyl having, in each case, 1 to 6 carbon atoms in the individual alkyl parts, or in each case optionally mono- or polysubstituted phenylalkyl or phenyl having, if appropriate, 1 to 4 carbon atoms in the straight-chain or branched alkyl part, the substituents in each case being in the phenyl part and being identical or different and suitable substituents in the phenyl part in each case being : halogen, cyano, nitro, in each case straight-chain or branched alkyl, alkoxy, alkylthio, alkylsulphinyl, alkylsulphonyl, halogenoalkyl, halogenoalkoxy, halogenoalkylthio, halogenoalkylsulphinyl or halogenoalkylsulphonyl in each case having 1 to 4 carbon atoms in the individual alkyl parts and, if appropriate, 1 to 9 identical or different halogen atoms,

$R^3$ represents in each case straight-chain or branched alkyl, alkenyl, alkinyl, halogenoalkyl or halogenoalkenyl, in each case having up to 8 carbon atoms and, if appropriate, having 1 to 17 halogen atoms, additionally represents in each case straight-chain or branched alkoxyalkyl or alkylthioalkyl in each case having 1 to 4 carbon atoms in the individual alkyl parts, cycloalkyl having 3 to 7 carbon atoms or optionally mono- or polysubstituted phenylalkyl, having 1 to 4 carbon atoms in the alkyl part and being straight-chain or branched in the alkyl part, the substitutents being in the phenyl part and being identical or different and suitable phenyl substituents being those mentioned in the case of $R^2$,

$R^4$ represents in each case straight-chain or branched alkyl, alkenyl, alkinyl, halogenoalkyl or halogenoalkenyl in each case having up to 8 carbon atoms and, if appropriate, 1 to 17 halogen atoms, additionally represents in each case straight-chain or branched alkoxyalkyl or alkylthioalkyl in each case having 1 to 4 carbon atoms in the individual alkyl parts, cycloalkyl having 3 to 7 carbon atoms, or optionally mono- or polysubstituted phenylalkyl, having 1 to 4 carbonatoms in the alkyl part and being straight-chain or branched in the alkyl part, the substituents being in the phenyl part and being identical or different and suitable phenyl substituents being those mentioned in the case of $R^2$, and in addition also represents hydrogen in the case where $R^3$ does not simultaneously represent unsubstituted alkyl, or

$R^3$ and $R^4$, together with the nitrogen atom to which they are bound, represent a 3- to 7-membered saturated heterocycle, which can contain up to 2 further heteroatoms, particularly nitrogen, oxygen and/or sulphur,

Ar represents in each case optionally mono- or polysubstituted phenyl, 2-pyridyl, 3-pyridyl or 4-pyridyl, the substituents being identical or different and suitable substituents in each case being : cyano, nitro, halogen, in each case straight-chain or branched alkyl, alkoxy or alkoxycarbonyl, in each case having 1 to 4 carbon atoms, in each case straight-chain or branched halogenoalkyl or halogenoalkoxy in each case having 1 to 4 carbon atoms and 1 to 9 identical or different halogen atoms, or a $-S(O)_p-R^6$ radical, and

n represents a number 0, 1 or 2, where

$R^6$ represents amino, and also in each case straight-chain or branched alkyl, alkylamino, dialkylamino or halogenoalkyl, in each case having up to 4 carbon atoms in the individual alkyl parts and, in the case of halogenoalkyl, having up to 9 identical or different halogen atoms, and

p represents a number 0, 1 or 2, characterised in that

(a) 5-halogeno-1-aryl-pyrazoles of the formula (II),

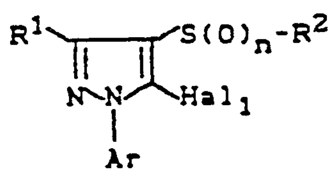

(II)

in which

$R^1$, $R^2$, n and Ar have the abovementioned meaning and

$Hal^1$ represents halogen, are reacted with amines of the formula (III),

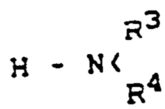

(III)

in which

$R^3$ and $R^4$ have the abovementioned meaning, if appropriate in the presence of a diluent and if appropriate in the presence of an acid acceptor, or in that

(b) 5-amino-1-aryl-pyrazoles of the formula

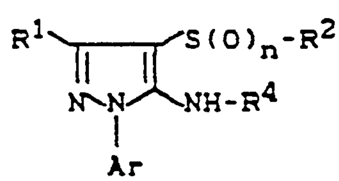

(IV)

in which

$R^1$, $R^2$, $R^4$, n and Ar have the abovementioned meaning, are reacted with alkylating agents of the formula (V)

$$R^3\text{-}E^1 \quad (V)$$

in which

$R^3$ has the abovementioned meaning and
$E^1$ represents an electron-withdrawing leaving group, if appropriate in the presence of a diluent, if appropriate in the presence of an acid acceptor and if appropriate in the presence of a catalyst ; or in that, to obtain 1-aryl-pyrazoles of the formula (Ia)

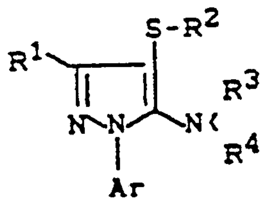
(Ia)

in which
$R^1$, $R^2$, $R^3$, $R^4$ and Ar have the abovementioned meaning,
(c) 4-unsubstituted 1-aryl-pyrazoles of the formula (VI),

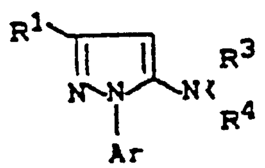
(VI)

in which
$R^1$, $R^3$, $R^4$ and Ar have the abovementioned meaning, are reacted with sulphenyl halides of the formula (VII),

$R^2$-S-$Hal^2$ (VII)

in which
$R^2$ has the abovementioned meaning and
$Hal^2$ represents halogen, if appropriate in the presence of a diluent and if appropriate in the presence of an acid acceptor ; or in that, to obtain 1-aryl-pyrazoles of the formula (Ib),

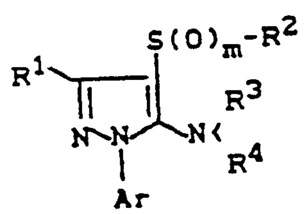
(Ib)

in which
$R^1$, $R^2$, $R^3$, $R^4$ and Ar have the abovementioned meaning and
m represents a number 1 or 2,
(d) the 1-aryl-pyrazoles of the formula (Ia),

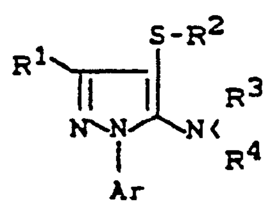
(Ia)

in which
$R^1$, $R^2$, $R^3$, $R^4$ and Ar have the abovementioned meaning, which are obtained by proces (a), (b) or (c) are reacted with oxidants of the formula (VIII),

$$R^5\text{-O-O-H} \quad \text{(VIII)}$$

in which
$R^5$ represents hydrogen or in each case optionally substituted alkanoyl or aroyl, if appropriate in the presence of a diluent, if appropriate in the presence of a catalyst and if appropriate in the presence of an acid acceptor.

4. Pesticides, characterised in that they contain at least one 1-aryl-pyrazole according to Claim 1.

5. Insecticidal and acaricidal agents, characterised in that they contain at least one 1-aryl-pyrazole according to Claim 1.

6. Process for combating insects and/or arachnida, characterised in that 1-aryl-pyrazoles according to Claim 1 are allowed to act on insects and/or arachnida and/or on their habitat.

7. Use of 1-aryl-pyrazoles according to Claim 1 for combating animal pests, particularly insects and/or arachnida.

8. Process for the preparation of pesticides, characterised in that 1-aryl-pyrazoles according to Claim 1 are mixed with extenders and/or surfactants.

## Revendications

1. 1-aryl-pyrazoles de formule générale (I) :

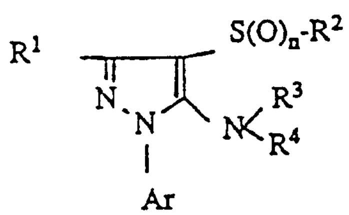
(I)

dans laquelle

$R^1$ représente un atome d'hydrogène, ou un groupe alkyle ou halogéno-alkyle, chaque fois liénaire ou ramifié et ayant 1 à 4 atomes de carbone et comportant éventuellement 1 à 9 atomes d'halogène, identiques ou différents,

$R^2$ représente un groupe alkyle, alcényle ou alcynyle, chacun linéaire ou ramifié, ayant chaque fois jusqu'à 8 atomes de carbone, un groupe cycloalkyle ayant 3 à 7 atomes de carbone, un groupe halogéno-alkyle ou halogéno-alcényle, chacun linéaire ou ramifié et ayant chacun jusqu'à 8 atomes de carbone et contenant jusqu'à 17 atomes d'halogéne identiques ou différents, un groupe alcoxyalkyle, alkylthioalkyle, alkylsulfinylalkyle ou alkylsulfonylalkyle, chacun linéaire ou ramifié et ayant chacun 1 à 6 atomes de carbone dans la partie alkyle, ou un groupe phénylalkyle ou phényl, dont la partie phényle est éventuellement substituée une ou plusieurs fois, de manière identique ou différente, et dont la partie alkyle éventuelle contient 1 à 4 atomes de carbone, en étant linéaire ou ramifiée, et l'on peut citer comme substituant dans la partie alkyle, à chaque fois : un atome d'halogène, un reste cyano, nitro, un reste alkyle, alcoxy, alkylthio, alkylsulfinyle, alkylsulfonyle, halogéno-alkyle, halogéno-alcoxy, halogéno-alkylthio, halogéno-alkylsulfinyle ou halogéno-alkylsulfonyle, chacun linéaire ou ramifié et ayant chacun 1 à 4 atomes de carbone dans les diverses parties alkyles et comportant éventuellement 1 à 9 atomes d'halogène, identiques ou différents,

$R^3$ représente un groupe alkyle, alcényle, alcynyle, halogénoalkyle ou halogéno-alcényle, chacun linéaire ou ramifié et ayant chacun jusqu'à 8 atomes de carbone et éventuellement 1 a 17 atomes d'halogène et, en outre, $R^3$ représente un groupe alcoxyalkyle ou alkylthioalkyle, chacun linéaire ou ramifié et ayant chacun 1 à 4 atomes de carbone dans les diverses parties alkyles, un groupe cycloalkyle ayant 3 a 7 atomes de carbone ou un groupe phénylalkyle dont la partie phényle est éventuellement substituée une ou plusieurs fois, de façon identique ou différente et dont la partie alkyle est linéaire ou ramifiée, le groupe comportant 1 à 4 atomes de carbone dans la partie alkyle, les substituants de la partie phényle étant ceux déjà cités pour $R^2$,

$R^4$ représente un groupe alkyle, alcényle, alcynyle, halogénoalkyle ou halogéno-alcényle, chacun linéaire

ou ramifié et ayant chacun jusqu'à 8 atomes de carbone et comportant éventuellement 1 a 17 atomes d'halogène et en outre un groupe alcoxyalkyle ou alkylthioalkyle, chacun linéaire ou ramifié et ayant chacun 1 à 4 atomes de carbone dans diverses parties alkyles, un groupe cycloalkyle ayant 3 a 7 atomes de carbone ou un groupe phénylalkyle, éventuellement substitué une ou plusieurs fois, de manière identique ou différente, dans la partie phényle et dont la partie alkyle est linéaire ou ramifiée et comporte 1 a 4 atomes de carbone, les substituants de la partie phényle étant ceux cités dans le cas de $R^2$, et, en outre, $R^4$ peut représenter aussi un atome d'hydrogène au cas ou $R^3$ ne représente pas simultanément un groupe alkyle non substitué, ou bien

$R^3$ et $R^4$ forment, avec l'atome d'azote auquel ils sont fixés, un hétérocycle saturé comportant 3 à 7 chaînons, qui peut contenir jusqu'a 2 autres hétéroatomes, en particulier de l'azote, de l'oxygène et/ou du soufre,

Ar représente un groupe phényle, 2-pyridyle, 3-pyridyle ou 4-pyridyle, chacun éventuellement substitué un ou plusieurs fois de manière identique ou différente, l'on peut citer à chaque fois comme substituant : un groupe cyano, nitro, halogéno, un groupe alkyle, alcoxy ou alcoxycarbonyle chacun linéaire ou ramifié et ayant chacun 1 à 4 atomes de carbone, un groupe halogéno-alkyle ou halogéno-alcolxy, chacun linéaire ou ramifié et ayant à chaque fois 1 à 4 atomes de carbone et 1 à 9 atomes d'halogène, identiques ou différents, ou à reste $-S(O)_p-R^6$, et

n représente un nombre valant 0, 1 ou 2, et

$R^6$ représente un groupe amino, ainsi qu'un groupe alkyle, alkylamino, dialkylamino ou halogéno-alkyle, chacun linéaire ou ramifié et ayant chacun jusqu'à 4 atomes de carbone dans la partie alkyle et, dans le cas d'un groupe halogénoalkyle, ayant jusqu'à 9 atomes d'halogène, identiques ou différents, et

p représente un nombre valant 0, 1 ou 2.

2. 1-aryl-pyrazoles selon la revendication 1, caractérisé en ce que, dans la formule générale (I),

$R^1$ représente un atome d'hydrogène, un groupe méthyle, éthyle, n- ou i- propyle et trifluorométhyle,

$R^2$ représente un groupe méthyle, éthyle, n- ou i-propyle, n-, i-, s- ou t-butyle, n- ou i-pentyle, n- ou i-hexyle, allyle, n- ou i-butényle, n- ou i-pentényle, propargyle, n- ou i-butynyle, cyclopropyle, cyclobutyle, cyclopentyle, cyclohexyle, chlorométhyle, difluorométhyle, difluorochlorométhyle, fluorodichlorométhyle, trifluorométhyle, pentafluoréthyle, pentachloréthyle, fluorotétrachloréthyle, difluorotrichloréthyle, trifluorodichloréthyle, trétrafluorochloréthyle, heptafluoropropyle, chloréthyle, brométhyle, chloropropyle, bromopropyle, dichlorométhyle, chlorofluorométhyle, trichlorométhyle, dichlorométhyle, trifluoréthyle, trifluorochloréthyle, tétrafluoréthyle, difluorochloréthyle, fluorodibromométhyle, difluorobromométhyle, fluorochlorobromométhyle, chlorallyle, fluorallyle, chlorobuténylе, fluorobuténylе, dichlorallyle, fluorochlorallyle, dichlorobuténylе, difluorallyle, bromallyle, un groupe méthoxyméthyle, méthoxyéthyle, méthoxypropyle, éthoxyméthyle, éthoxyéthyle, méthylthiométhyle, méthylsulfinylméthyle, méthylsulfonyléthyle, méthylthioéthyle, méthylthiopropyle, méthylsulfinyléthyle, méthylsulfonylméthyle, ou un groupe phényle, benzyle ou phényléthyle, chacun éventuellement substitué 1 à 3 fois de manière identique ou différente, et l'on peut citer à chaque fois comme substituants de la partie phényle : un atome de fluor, de chlore, de brome, d'iode, un groupe cyano, nitro, méthyle, éthyle, méthoxy, méthylthio, trifluorométhyle, méthylsulfinyle, méthylsulfonyle, trifluorométhoxy, trifluorométhylthio, trifluorométhylsulfinyle, ou trifluorométhylsulfonyle,

$R^3$ représente un groupe méthyle, éthyle, n- ou i-propyle, n-, i-, s- ou t-butyle, n- ou i-pentyle, n- ou i-hexyle, un groupe allyle, n- ou i-buténylе, n- ou i-penténylе, propargyle, n- ou i-butynyle, cyclopropyle, cyclobutyle, cyclopentyle, cyclohexyle, chlorométhyle, difluorométhyle, difluorochlorométhyle, fluorodichlorométhyle, trifluorométhyle, pentafluoréthyle, pentachloréhtyle, fluorotétrachloréthyle, difluorotrichloréthyle, trifluorodichloréthyle, tétrafluorochloréthyle, heptafluoropropyle, chloréthyle, brométhyle, chloropropyle, bromopropyle, dichlorométhyle, chlorofluorométhyle, trichlorométhyle, trifluoréthyle, trifluorochloréthyle, tétrafluoréthyle, difluorochloréthyle, fluorodibromométhyle, difluorobromométhyle, fluorochlorobromométhyle, chlorallyle, fluorallyle, chlorobuténylе, fluorobuténylе, dichlorallyle, fluorochlorallyle, dichlorobuténylе, difluorallyle, bromallyle, un groupe méthoxyméthyle, méthoxyéthyle, méthoxypropyle, éthoxyméthyle, éthoxyéthyle, méthylthiométhyle, méthylthioéthyle, méthylthiopropyle, éthylthiométhyle, éthylthioéthyle ou un groupe benzyle ou phényléthyle, dont la partie phényle est éventuellement substituée 1 a 3 fois de manière identique ou différente, et l'on peut citer comme substituants du groupe phényle ceux cités à propos de $R^2$,

$R^4$ représente un groupe méthyle, éthyle, n- ou i-propyle, n-, i-, s- ou t-butyle, n- ou i-pentyle, n- ou i-hexyle, un groupe allyle, n- ou i-buténylе, n- ou i-penténylе, propargyle, n- ou i-butynyle, cyclopropyle, cyclobutyle, cyclopentyle, -cyclohexyle, chlorométhyle, difluorométhyle, difluorochlorométhyle, fluorodichlorométhyle, trifluorométhyle, pentafluoréthyle, pentachloréhtyle, fluorotétrachloréthyle, difluorotrichloréthyle, trifluorodichloréthyle, tétrafluorochloréthyle, heptafluoropropyle, chloréthyle, brométhyle, chloropropyle, bromopropyle, dichlorométhyle, chlorofluorométhyle, trichlorométhyle, trifluoréthyle, trifluorochloréthyle, tétrafluoréthyle, difluorochloréthyle, fluorodibromométhyle, difluorobromométhyle, fluorochlorobromométhyle, chlorallyle, fluorallyle, chlorobuténylе, fluorobuténylе, dichlorallyle, fluorochlorallyle, dichlorobuténylе, difluorallyle, bromallyle, un groupe méthoxyméthyle, méthoxyéthyle, méthoxypropyle, éthoxyméthyle, éthoxyéthyle, méthylthiométhyle,

méthylthioéthyle, méthylthiopropyle, éthylthiométhyle, éthylthioéthyle ou un groupe benzyle ou phényléthyle, éventuellement substitué 1 a 3 fois, de manière identique ou différente, dans la partie phényle, les substituants de la partie phényle étant ceux cités pour $R^2$, et, en outre, $R^4$ peut représenter également un atome d'hydrogène au cas ou $R^3$ ne représente pas en même temps un groupe alkyle non substitué, ou bien

$R^3$ et $R^4$ forment, avec l'atome d'azote auquel ils sont fixés, un groupe pyrrolidinyle, pipéridinyle, perhydroazépinyle ou morpholinyle,

Ar représente un groupe phényle, éventuellement substitué 1 a 5 fois, de manière identique ou différente, ou un groupe 2-pyridyle éventuellement substitué 1 a 4 fois, de manière identique ou différente, et l'on peut alors citer comme substituant a chaque fois : un groupe cyano, nitro, fluoro, chloro, bromo, iodo, méthyle, éthyle, n- et i-propyle, n-, i-, s- et t-butyle, méthoxy, éthoxy, méthoxycarbonyle, éthoxycarbonyle, trifluorométhyle, trichlorométhyle, dichlorofluorométhyle, difluorochlorométhyle, chlorométhyle, dichlorométhyle, difluorométhyle, pentafluoroéthyle, tétrafluoréthyle, trifluorochloréthyle, trifluoréthyle, difluorodichloréthyle, trifluorodichloréthyle, pentachloréthyle, trifluorométhoxy, trichlorométhoxy, dichlorofluorométhoxy, difluorochlorométhoxy, chlorométhoxy, dichlorométhoxy, difluorométhoxy, pentafluoréthoxy, tétrafluoréthoxy, trifluorochloréthoxy, trifluoréthoxy, difluorodichloréthoxy, trifluorodichloréthoxy, pentachloréthoxy ou un reste $-S(O)_p-R^6$, et

n représente un nombre valant 0, 1 ou 2,

$R^6$ représente un groupe amino, méthylamino, éthylamino, diméthylamino, diéthylamino, fluorodichlorométhyle, difluorométhyle, tétrafluoréthyle, trifluorochloréthyle, trichlorométhyle, trichloréthyle, trifluorométhyle, méthyle ou éthyle, et

p représente un nombre valant 0, 1 ou 2.

3. Procédé pour préparer des 1-aryl-pyrazoles de formule générale (I) :

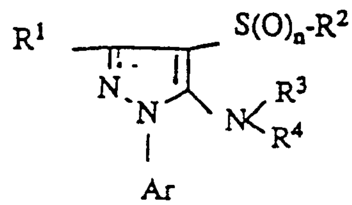

(I)

dans laquelle

$R^1$ représente un atome d'hydrogène, ou un groupe alkyle ou halogéno-alkyle, chaque fois linéaire ou ramifié et ayant 1 à 4 atomes de carbone et comportant éventuellement 1 à 9 atomes d'halogène, identiques ou différents,

$R^2$ représente un groupe alkyle, alcényle ou alcynyle, chacun linéaire ou ramifié ayant chaque fois jusqu'à 8 atomes de carbone, un groupe cycloalkyle ayant 3 à 7 atomes de carbone, un groupe halogéno-alkyle ou halogéno-alcényle, chacun linéaire ou ramifié et ayant chacun jusqu'à 8 atomes de carbone et contenant jusqu'à 17 atomes d'halogène identiques ou différents, un groupe alcoxyalkyle, alkylthioalkyle, alkylsulfinylalkyle ou alkylsulfonylalkyle, chacun linéaire ou ramifié et ayant chacun 1 a 6 atomes de carbone dans la partie alkyle, ou un groupe phénylalkyle ou phényl, dont la partie phényle est éventuellement substituée une ou plusieurs fois, de manière identique ou différente, et dont la partie alkyle éventuelle contient 1 a 4 atomes de carbone, en étant linéaire ou ramifiée, et l'on peut citer comme substituant dans la partie alkyle, à chaque fois : un atome d'halogène, un reste cyano, nitro, un reste alkyle, alcoxy, alkylthio, alkylsulfinyle, alkylsulfonyle, halogéno-alkyle, halogéno-alcoxy, halogéno-alkylthio, halogéno-alkylsulfinyle ou halogéno-alkylsulfonyle, chacun linéaire ou ramifié et ayant chacun 1 à 4 atomes de carbone dans les diverses parties alkyles et comportant éventuellement 1 à 9 atomes d'halogène, identiques ou différents,

$R^3$ représente un groupe alkyle, alcényle, alcynyle, halogénoalkyle ou halogéno-alcényle, chacun linéaire ou ramifié et ayant chacun jusqu'à 8 atomes de carbone et éventuellement 1 à 17 atomes d'halogène et, en outre, $R^3$ représente un groupe alcoxyalkyle ou alkylthioalkyle, chacun linéaire ou ramifié et ayant chacun 1 à 4 atomes de carbone dans les diverses parties alkyles, un groupe cycloalkyle ayant 3 à 7 atomes de carbone ou un groupe phénylalkyle dont la partie phényle est éventuellement substituée une ou plusieurs fois, de façon identique ou différente et dont la partie alkyle est linéaire ou ramifiée, le groupe comportant 1 à 4 atomes de carbone dans la partie alkyle, les substituants de la partie phényle étant ceux déjà cités pour $R^2$,

$R^4$ représente un groupe alkyle, alcényle, alcynyle, halogénoalkyle ou halogéno-alcényle, chacun linéaire ou ramifié et ayant chacun jusqu'à 8 atomes de carbone et comportant éventuellement 1 à 17 atomes d'halogène et en outre un groupe alcoxyalkyle ou alkylthioalkyle, chacun linéaire ou ramifié et ayant chacun 1 à 4 atomes de carbone dans diverses parties alkyles, un groupe cycloalkyle ayant 3 à 7 atomes de carbone ou un

groupe phénylalkyle, éventuellement substitué une ou plusieurs fois, de manière identique ou différente, dans la partie phényle et dont la partie alkyle est linéaire ou ramifiée et comporte 1 à 4 atomes de carbone, les substituants de la partie phényle étant ceux cités dans le cas de $R^2$, et, en outre, $R^4$ peut représenter aussi un atome d'hydrogène au cas où $R^3$ ne représente pas simultanément un groupe alkyle non substitué, ou bien

$R^3$ et $R^4$ forment, avec l'atome d'azote auquel ils sont fixés, un hétérocycle saturé comportant 3 à 7 chaînons, qui peut contenir jusqu'a 2 autres hétéroatomes, en particulier de l'azote, de l'oxygène et/ou du soufre,

Ar représente un groupe phényle, 2-pyridyle, 3-pyridyle ou 4-pyridyle, chacun éventuellement substitué un ou plusieurs fois de manière identique ou différente, l'on peut citer à chaque fois comme substituant : un groupe cyano, nitro, halogéno, un groupe alkyle, alcoxy ou alcoxycarbonyle chacun linéaire ou ramifié et ayant chacun 1 à 4 atomes de carbone, un groupe halogéno-alkyle ou halogéno-alcolxy, chacun linéaire ou ramifié et ayant à chaque fois 1 à 4 atomes de carbone et 1 à 9 atomes d'halogène, identiques ou différents, ou a reste $-S(O)_p-R^6$, et

n représente un nombre valant 0, 1 ou 2, et

$R^6$ représente un groupe amino, ainsi qu'un groupe alkyle, alkylamino, dialkylamino ou halogéno-alkyle, chacun linéaire ou ramifié et ayant chacun jusqu'à 4 atomes de carbone dans la partie alkyle et, dans le cas d'un groupe halogénoalkyle, ayant jusqu'à 9 atomes d'halogène, identiques ou différents, et

p représente un nombre valant 0, 1 ou 2, procédé caractérisé en ce que :

(a) on fait réagir des 5-halogéno-1-aryl-pyrazoles de formule (II) :

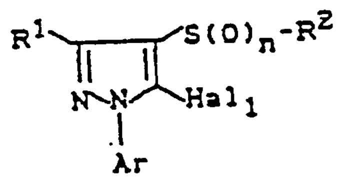

(II)

dans laquelle

$R^1$, $R^2$, n et Ar ont le sens indiqué ci-dessus, et $Hal_1$ représente un atome d'halogène, avec des amines de formule (III) :

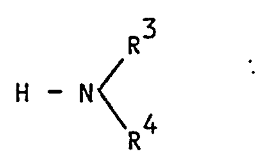

(III)

dans laquelle

$R^3$ et $R^4$ ont le sens indiqué ci-dessus, en opérant éventuellement en présence d'un diluant et éventuellement en présence d'un agent de fixation des acides, ou bien

(b) on fait réagir des 5-amino-aryl-pyrazoles de formule :

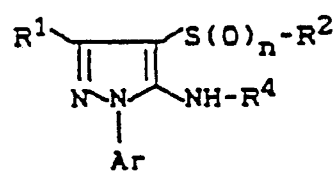

(IV)

dans laquelle

$R^1$, $R^2$, $R^4$, n et Ar ont le sens indiqué ci-dessus, avec des agents d'alkylation de formule (V) :

$$R^3\text{-}E^1 \quad (V)$$

dans laquelle

$R^3$ a le sens indiqué ci-dessus, et

$E^1$ représente un groupe partant attracteur d'électrons, en opérant éventuellement en présence d'un

diluant, éventuellement en présence d'un agent de fixation des acides et éventuellement en présence d'un catalyseur ; ou bien, pour obtenir des 1-aryl-pyrazoles de formule (Ia) :

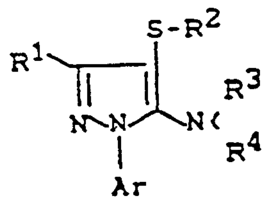

(Ia)

dans laquelle
$R^1$, $R^2$, $R^3$, $R^4$ et Ar ont le sens indiqué ci-dessus,
(c) on fait réagir des 1-aryl-pyrazoles, non substitués en position 4 et répondant à la formule (VI) :

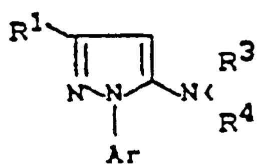

(VI)

dans laquelle
$R^1$, $R^3$, $R^4$ et Ar ont le sens indiqué ci-dessus, avec des halogénures de sulfényle de formule (VII) :

$$R^2\text{-S-Hal}^2 \quad \text{(VII)}$$

dans laquelle
$R^2$ a le sens précité, et
$Hal^2$ représente un atome d'halogène, en opérant éventuellement en présence d'un diluant et éventuellement en présence d'un agent de fixation des acides ; ou bien, pour obtenir des 1-aryl-pyrazoles de formule (Ib) :

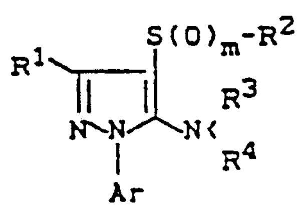

(Ib)

dans laquelle
$R^1$, $R^2$, $R^3$, $R^4$ et Ar ont le sens indiqué ci-dessus, et
m est un nombre valant 1 ou 2,
(d) on fait réagir les 1-aryl-pyrazoles de formule (Ia) :

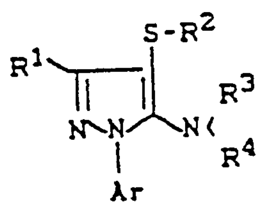

(Ia)

dans laquelle
$R^1$, $R^2$, $R^3$, $R^4$ et Ar ont le sens indiqué ci-dessus, que l'on peut obtenir selon le procédé (a), (b), ou (c) avec des oxydants de formule (VIII) :

$R^5$-O-O-H (VIII)

dans laquelle
$R^5$ représente un atome d'hydrogène ou un groupe alcanoyle ou aroyle, chacun éventuellement substitué, en opérant éventuellement en présence d'un diluant, éventuellement en présence d'un catalyseur et éventuellement en présence d'un agent de fixation des acides.

4. Produit pour lutter contre les parasites, produit caractérisé en ce qu'il contient au moins un 1-aryl-pyrazole selon la revendication 1.

5. Insecticides et acaricides, caractérisés en ce qu'ils contiennent au moins un 1-aryl-pyrazole selon la revendication 1.

6. Procédé pour combattre les insectes et/ou les arachnides, caractérisé en ce qu'on laisse agir sur les insectes et/ou sur les arachnides et/ou sur leur biotope ou espace vital des 1-aryl-pyrazoles selon la revendication 1.

7. Application des 1-aryl-pyrazoles selon la revendication 1, pour lutter contre des parasites animaux, en particulier des insectes et/ou des arachnides.

8. Procédé pour préparer des pesticides, caractérisé en ce qu'on mélange des 1-aryl-pyrazoles selon la revendication 1, avec des agents d'allongement et/ou des agents tensioactifs.